# EUROPEAN PATENT APPLICATION

(11) **EP 0 758 680 A2**
(43) Date of publication of application: **19.02.1997**
(21) Application number: 96104885.7
(22) Date of filing: 27.03.1996
(51) Int. Cl.: C12N 11/12, C02F 3/10, C02F 3/12, C02F 3/28, C02F 3/30

(54) **Carrier for immobilizing microorganisms, and method for converting nitrogen compounds in a liquid using the same**

(30) Priority: 27.03.1995 JP 19950094493
(71) Applicant: Matsumura, Masatoshi, Tsukuba-shi, Ibaragi 305 (JP); Biomaterial Co., Ltd., Fukui-shi, Fukui (JP)
(72) Inventor: Matsumura, Masatoshi, Tsukuba-shi, Ibaragi (JP); Fujii, Naoyuki, Fukui-shi, Fukui (JP)
(74) Representative: VOSSIUS & PARTNER

(57) **Abstract**

A carrier for immobilizing microorganisms, comprising a porous cellulose derivative or a porous cellulose, which has a good affinity for the microorganisms, can be applied to any liquid treatment tank without limiting use sites, and does not require a plant investment for immobilization of the microorganisms, and a method of converting nitrogen compounds in a liquid to be treated such as potable water or wastewater, by introducing the carrier together with the liquid to be treated into a liquid treatment tank such as a nitrification treatment tank or a denitrification treatment tank, thereby converting the nitrogen compounds contained in the liquid to be treated into harmless nitrogen compounds, are disclosed.

## Description

The present invention relates to a carrier for immobilizing microorganisms, which comprises a porous cellulose derivative, and to a method of converting nitrogen compounds contained in a liquid using the same. More specifically, the invention relates to a carrier for immobilizing microorganisms, which comprises a porous cellulose derivative or a porous cellulose, which controls biodegradation due to microorganisms by reacting a wide variety of compounds with cellulose, and to a method which can convert harmful nitrogen compounds in a liquid contained in potable water or wastewater into harmless ones by introducing the carrier for immobilizing microorganisms in a liquid treatment tank such as a nitrification treatment tank or a denitrification treatment tank.

Recently, with the increase in the consumption of nitrogen fertilizers, the contamination of groundwater and the like with nitrogen compounds has been proceeded. Nitric acid is a substance that generates the contamination of groundwater. There was a case in European and American countries that babies and little children who drunk the water containing nitrate died of anemic blue baby syndrome. In this case, gastric juice in the stomach of babies and little children are not acidic. That is, nitric acid converts into nitrous acid due to microorganisms in stomach, and nitrite bonds to hemoglobins in blood. As a result, since the supply of oxygen is insufficient, the babies and little children died.

World Health Organization (WHO) determines the concentration of nitrate nitrogen to be 10 ppm as a standard. However, groundwater in European and American countries actually far exceeds this standard of 10 ppm. For example, in Denmark the number of wells detecting a concentration of the nitrate nitrogen of 50 ppm occupies about 8% of the all. Further, nitrate nitrogen at a high concentration of 150 ppm is detected from groundwater in the United States of America. Furthermore, groundwater in some cultivated fields in Japan is found to be contained nitrate nitrogen in an amount exceeding the standard of 10 ppm, and the Ministry of Public Welfare and the Environment Agency in Japan tightened up the investigation on concentration of nitrate nitrogen as an item of the environmental observation.

Further, in the treatment of wastewater such as sewage, the eutrophication of lakes and rivers also proceeds. Despite that improvement and amplification of sewerage facilities are made, it is the existing state that problems are not fundamentally solved. The treatment employed is an activated sludge treatment, which can decrease a biochemical oxygen demand (BOD) attributable to microorganisms, but cannot remove the nutrients such as nitrogen compounds, phosphorus, or the like, which were derived from organic compounds contained in the wastewater. As a result, the lakes and rivers, into which the nutrients are released, are the state that plant planktons are liable to multiply, and the eutrophication again proceeds, resulting in the progress of the contamination of lakes and rivers.

Various methods are applied to a drainage of groundwater, wastewater and the like to remove ammonia nitrogen, nitrate nitrogen and nitrite nitrogen which are converted from harmful organic nitrogen. One of the methods is a biochemical denitrification method. This method basically conducts a treatment in a nitrification treatment tank or a denitrification treatment tank using nitrifying bacteria or denitrifying bacteria which present in an activated sludge. That is, the nitrification treatment tank can convert organic nitrogen into ammonia nitrogen, and can also convert the ammonia nitrogen into nitrate nitrogen or nitrite nitrogen using nitrifying bacteria having a slow multiplication rate. The nitrate nitrogen and nitrite nitrogen which were converted and formed from the ammonia nitrogen in the nitrification treatment tank are generally further converted into nitrogen using denitrifying bacteria in the denitrification treatment tank, into which a hydrogen donor was add, to release the nitrogen into atmosphere. Thus, nitrogen compounds are removed from groundwater and wastewater. To attempt an increase in the treatment capacity, it is proposed to introduce a polyurethane foam or a carrier for immobilizing microorganisms prepared from jelly-like polyethylene glycol derivatives as disclosed in JP-A-5-023684 into the nitrification treatment tank (The term "JP-A" used herein means an "unexamined published Japanese patent application"). Further, to produce the useful material such as citric acid, it is also proposed to use a cellulose foam as the carrier for immobilizing microorganisms.

However, the conventional methods have the following problems. The method of using a carrier prepared from the polyethylene glycol derivatives as disclosed in JP-A-5-023684 must previously immobilize microorganisms onto the carrier. This requires an apparatus for immobilizing microorganisms, resulting in an investment to a plant. The carrier prepared from the polyethylene glycol derivatives can immobilize nitrifying bacteria thereon in the nitrification treatment tank under aerobic conditions, but cannot immobilize denitrifying bacteria thereon in the denitrification treatment tank under anaerobic conditions. Thus, the conventional method lacks in the wide use.

When a polyurethane foam is used, in the sense that the polyurethane foam can be used in the denitrification treatment tank, it can overcome the disadvantage of the polyethylene glycol derivatives, but the polyurethane foam does not have a sufficient affinity for the above-mentioned microorganisms. Therefore, the maximum nitrification rate of the polyurethane foam carrier to be used in the nitrification treatment tank is low as 130 (mg-N/I-carrier/hr), so that it is difficult to expect further increase in the treatment capacity. In addition, since the polyurethane foam after used is mere synthetic resin, it requires a consideration about the disposal of the polyurethane foam.

Further, the cellulose foam has markedly a short life under the environment where cellulose degradation activity is high, and does not withstand a use for several months. Thus, the cellulose foam is not suited to use in various purposes.

Accordingly, one object of the present invention is to provide a carrier for immobilizing microorganisms which is excellent in immobilization of microorganisms in high density, and can be used in any liquid treatment tanks without limiting use sites thereof, and does not require a plant investment for immobilizing microorganisms.

Another object of the present invention is to provide a method for converting nitrogen compounds contained in a liquid such as potable water or wastewater, which can increase the treatment capacity when the carrier is used in a liquid treatment tank such as a nitrification treatment tank or a denitrification tank.

Further object of the present invention is to provide a carrier for immobilizing microorganisms, which can be easily disposed by incinerating or land filling to degrade the carrier even after used in the liquid treatment tank.

The problems involved in the prior art can be overcome by the present invention which relates to a carrier for immobilizing microorganisms, which comprises a porous cellulose or a porous cellulose derivative.

The present invention also relates to a method of converting nitrogen compounds in a liquid to be treated, which comprises: introducing the carrier for immobilizing microorganisms together with the liquid to be treated into a liquid treatment tank under aerobic conditions or anaerobic conditions; and stirring the resulting mixture, thereby converting nitrogen compounds contained in the liquid to be treated.

In a preferred embodiment of the present invention, the carrier for immobilizing microorganisms comprises a porous cellulose derivative obtained by reacting at least one member selected from the group consisting of an epoxy compound having an epoxy group, an N-methylol compound, an imidazolidinone compound, an aldehyde compound having an aldehyde group, an acetal compound, an active vinyl compound, an aziridinyl compound, a compound having a carboxyl group, a compound having an acyl group, a quaternary ammonium compound, an amidophosphazene compound, and a compound having an isocyanate group with cellulose.

In a further preferred embodiment of the present invention, the carrier comprises a porous cellulose obtained by coating cellulose with a compound which is obtained by reacting an epoxy compound having an epoxy group with a polyamine compound.
Figure 1 is an explanatory drawing of a tower-like gas lift-type liquid treatment tank;
Figure 2 is an explanatory drawing of a box-like gas lift-type liquid treatment tank;
Figure 3 is an explanatory drawing of a down-flow expand-type liquid treatment tank;
Figure 4 is an explanatory drawing of an ejector-type liquid treatment tank;
Figure 5 is an explanatory drawing of another example of an ejector-type liquid treatment tank;
Figure 6 is an explanatory drawing of a liquid current jet device;
Figure 7 is an explanatory drawing of a liquid treatment tank which is used as a nitrification tank;
Figure 8 is an explanatory drawing of a liquid treatment tank which is used as a denitrification tank;
Figure 9 is an explanatory drawing of a liquid treatment tank in which a nitrification tank and a denitrification tank are concurrently used;
Figure 10 is an explanatory drawing of another example of a liquid treatment tank in which a nitrification tank and a denitrification tank are concurrently used;
Figure 11 is a graph showing a measured residual amount of ammonia nitrogen (NH₄-N) in a liquid which was subjected to nitrification treatment using the box-like gas lift-type liquid treatment tank;
Figure 12 is a graph showing a measured residual amount of ammonia nitrogen (NH₄-N) in a liquid which was subjected to nitrification treatment using a carrier having a pore diameter of 50 µm;
Figure 13 is a graph showing a measured residual amount of ammonia nitrogen (NH₄-N) in a nitrification tank in which nitrification treatment was conducted using a carrier having a pore diameter of 500 µm;
Figure 14 is a graph showing a measured residual amount of ammonia nitrogen (NH₄-N) in a liquid which was subjected to nitrification treatment using a carrier having a pore diameter of 1,260 µm;
Figure 15 is a graph showing a measured residual amount of nitrate ammonia (NO₃-N) in a liquid which was subjected to denitrification treatment using the tower-like gas lift-type liquid treatment tank;
Figure 16 is a graph showing a measured residual amount of nitrate ammonia (NO₃-N) in a liquid which was subjected to denitrification treatment using the down-flow expand-type liquid treatment tank; and
Figure 17 is a graph showing a measured residual amount of nitrate ammonia (NO₃-N) in a liquid which was subjected to denitrification treatment using the ejector-type liquid treatment tank.

The porous cellulose derivative which can be used in the present invention is a compound having a functional group reactive to a hydroxyl group of glucose which constitutes cellulose. The compound has a network structure and is one to which crosslinking is subjected between the molecules. The compound can be obtained by reacting at least one member selected from the group consisting of an epoxy compound having an epoxy group, an N-methylol compound, an imidazolidinone compound, an aldehyde compound having an aldehyde group, an acetal compound, an active vinyl compound, an aziridinyl compound, a compound having a carboxyl group, a compound having an acyl group, a quaternary ammonium compound, an amidophosphazene compound, and a compound having an isocyanate group with cellulose.

The porous cellulose derivative according to the invention, obtained by reacting an epoxy compound having an epoxy group with cellulose in the present invention is that a hydroxyl group in glucose which constitutes cellulose reacts with an epoxy group in the epoxy compound, and the epoxy is ring-opened, thereby crosslinking the glucose which constitutes the cellulose. The epoxy compound is reacted in an amount of from 3 to 60% by weight, preferably from 5 to 30% by weight, and more preferably from 20 to 30% by weight, based on the weight of the cellulose. If the amount of the epoxy compound is less than 3% by weight, degradation of cellulose with microorganisms is accelerated, and as a result, the carrier cannot be used over a long period of time. On the other hand, if the amount of the epoxy compound exceeds 60% by weight, the cellulose loses the inherent properties as the carrier and also is liable to disintegrate. For those reasons, it is desirable that the epoxy compound be used within the above-described range.

A specific method for obtaining the porous cellulose derivative by crosslinking cellulose with the epoxy compound is that the cellulose is immersed in a solution prepared by dissolving the epoxy compound into a solvent or in a dispersion prepared by dispersing the epoxy compound into a solvent, and reaction is conducted at room temperature or under heating.

The solvent which can be used is a polar solvent such as water, alcohols, ketones or ethers. Those solvents can be used alone or as mixtures thereof. The preferred examples of the solvent include water, ethanol, methanol, isopropyl alcohol, dimethylsulfoxide, and N,N-dimethylformamide. Reaction can be conducted using those solvents at a temperature of from 10 to 200 °C when humidity is from 10 to 60% RH, and at a temperature of from 50 to 140°C when humidity is from 60 to 100% RH. The reaction time can be controlled by, in particular, the shape of the cellulose, and is preferably from about 0.5 to 200 minutes, and more preferably from 30 to 60 minutes. If required and necessary, the reaction product can be aged by allowing it to leave at room temperature in order to complete the reaction between the epoxy compound and the cellulose.

In this reaction, it is possible to proceed the reaction in the presence of a catalyst. Examples of the catalyst include sodium hydroxide (NaOH), a Lewis acid such as trifluoroboron ammonia complex, zinc borofluoride (Zn(BF₄)₂), and tin tetrachloride (SnCl₄). The amount of the catalyst to be added is preferably from 1 to 10% by weight based on the weight of the epoxy compound when sodium hydroxide or a Lewis acid such as trifluoroboron ammonia complex is used. The catalyst can previously be added to the solvent or can be added to the solution or dispersion when the cellulose is immersed in the solution or dispersion.

The porous cellulose derivative used in the present invention can be obtained using the epoxy compounds represented by the following formulae (2), (3), (4), (5) and (6). The preferred use embodiment of those epoxy compounds is that the epoxy compound having an epoxy group is at least one member selected from the formula (2), the formula (3), the formula (4), the formula (5) and the formula (6). 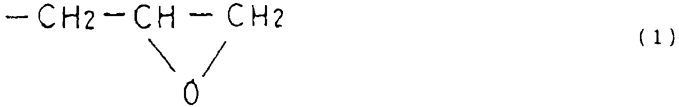 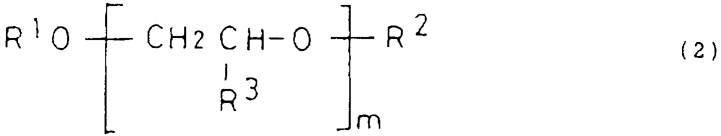 wherein m represents an integer of from 0 to 50; R¹ and R² may be the same or different and represent a hydrogen atom, a methyl group or a group of formula (1) above and R³ represents a hydrogen atom or a methyl group; with the proviso that at least one of R¹ and R² represents a group of formula (1); 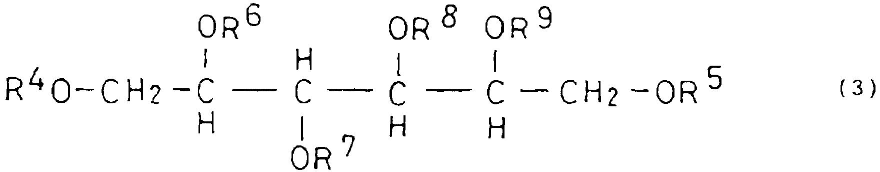 wherein R⁴, R⁵, R⁶, R⁷, R⁸ and R⁹ may be the same or different and represent a hydrogen atom, a phenyl group, a methyl group or a group of formula (1) above, with the proviso that at least one of R⁴, R⁵, R⁶, R⁷, R⁸ and R⁹ represents a group of formula (1); 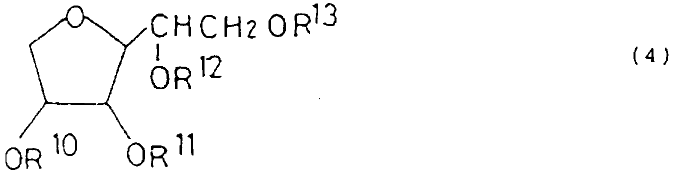 wherein R¹⁰, R¹¹, R¹² and R¹³ may be the same or different, and represent a hydrogen atom, a phenyl group, a methyl group or a group of formula (1) above, with the proviso that at least one of R¹⁰, R¹¹, R¹² and R¹³ represents a group of formula (1); 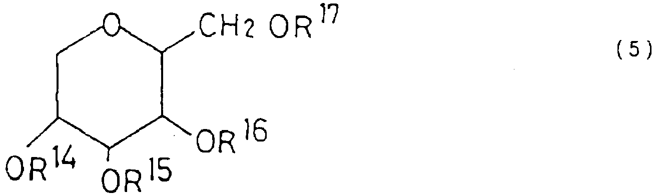 wherein R¹⁴, R¹⁵, R¹⁶ and R¹⁷ may be the same or different and represent a hydrogen atom, a phenyl group, a methyl group or a group of formula (1) above, with the proviso that at least one of R¹⁴, R¹⁵, R¹⁶ and R¹⁷ represents a group of formula (1); 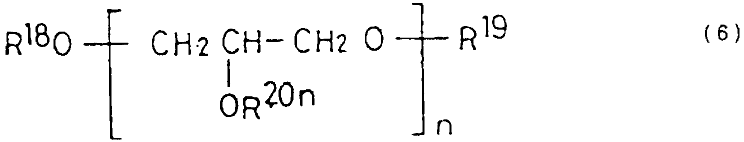 wherein n represents an integer of from 1 to 10; R¹⁸, R¹⁹ and R²⁰ⁿ may be the same or different, and represent a hydrogen atom, a phenyl group, a methyl group or a group of formula (1) above, with the proviso that at least one of R¹⁸, R¹⁹ and R²⁰ⁿ represents a group of formula (1).

It is preferred for the epoxy compound having the above formula (2), (3), (4), (5) or (6) as the basic structure to have at least two of the formula (1) above. Due to that the epoxy compound has a plurality of the formula (1), the epoxy groups bond to hydroxyl groups in the glucose which constitutes the cellulose, and this enables to more effectively control degradation of the cellulose with microorganisms.

The preferred examples of the epoxy compound are the epoxy compound represented by the formula (2) wherein both R¹ and R² represent the formula (1), the epoxy compound represented by the formula (3) wherein at least two of R⁴, R⁵, R⁶, R⁷, R⁸ and R⁹ represent the formula (1), the epoxy compound represented by the formula (4) wherein at least two of R¹⁰, R¹¹, R¹² and R¹³ represent the formula (1), the epoxy compound represented by the formula (5) wherein at least two of R¹⁴, R¹⁵, R¹⁶ and R¹⁷ represent the formula (1), and the epoxy compound represented by the formula (6) wherein at least two of R¹⁸, R¹⁹ and R²⁰ⁿ represent the formula (1). In particular, it is preferred in the epoxy compounds represented by the formulae (3), (4), (5) and (6) that the positions to which the formula (1) is bonded are far apart. For example, it is preferred in the epoxy compound represented by the formula (3) that R⁴ and R⁵ are the group of the formula (1).

R²⁰ⁿ in the formula (6) can be the same or different when the bracket portion is repeated. For example, when n is 2, the bracket portion is repeated twice, and as a result, the first R²⁰ⁿ is R²⁰¹ and the second R²⁰ⁿ is R²⁰². Those R²⁰ⁿ(R²⁰¹ and R²⁰²) may be the same or different.

More preferred examples of the epoxy compound include the epoxy compound represented by the formula (2) wherein m is 0, R¹ represents the formula (1), and R² represents a hydrogen atom; the epoxy compound represented by the formula (2) wherein m is 5, R¹ represents the formula (1), R² represents a phenyl group, and R³ represents a hydrogen atom; the epoxy compound represented by the formula (2) wherein m is 1, R¹ and R² each represents the formula (1), and R³ represents a hydrogen atom; the epoxy compound represented by the formula (3) wherein R⁴, R⁵, R⁶ and R⁹ each represents the formula (1), and R⁷ and R⁸ each represents a hydrogen atom; and the epoxy compound represented by the formula (6) wherein n is 1, R¹⁸ and R¹⁹ each represents the formula (1), and R²⁰ⁿ represents a hydrogen atom.

Specific examples of the epoxy compound include glycidol, glycerol diglycidyl ether, glycerol triglycidyl ether, polyglycerol polyglycidyl ether, polyethylene glycol diglycidyl ether, ethylene glycol diglycidyl ether, polypropylene glycol diglycidyl ether, sorbitol polyglycidyl ether, sorbitan diglycidyl ether, bis-(2,3-epoxycyclopentyl)-ether, vinylcyclohexanedioxide butadienediepoxide, 1,2-bis-(2,3-epoxy-2-methylpropoxy)-ethane, and 1,1,3-tris-(2,3-epoxy-propoxy)-butane. Those compounds can be used alone or as mixtures thereof.

The porous cellulose derivatives obtained by reacting at least one member selected from the group consisting of an N-methylol compound, an imidazolidinone compound, an aldehyde compound having an aldehyde group, an acetal compound, an active vinyl compound, an aziridinyl compound, a compound having a carboxyl group, a compound having an acyl group, a quaternary ammonium compound, an amidophosphazene compound, and a compound having an isocyanate group with cellulose are functional groups of those compounds, similarly to the epoxy compound, react with hydroxyl groups of glucose which constitutes cellulose to cross link glucoses each other.

Examples of the N-methylol compound include dimethyl urea (DMU), methylated trimethylolmelamine (MTMM), dimethylolethylene urea (DMEU), dimethylolmethyl triazone (DMTr), dimethylolethyl triazone, dimethylolhydroxyethyl triazone, methylated dimethylol urone (DMUr), hexamethylol melamine (HMM), dimethylolpropylene urea (DMPU), dimethyloldihydroxyethylene urea (DMDHEU), tetramethylolacetylene diurea (TMADU), 4-methoxy-5-dimethylpropylene urea dimethylol compound (4MO, 5DM, PU), dimethylolmethyl carbamate (DMAC), dimethylolethyl carbamate, dimethylolhydroxyethyl carbamate, dimethylolhydroxyisopropylcarbamate,dimethyloldimethoxyethylene urea, dimethylolbutylene urea, dimethylol-5-hydroxypropylene urea, dimethylol urone, and tetramethylolethylene bistriazone. Those compounds can be used alone or as mixtures thereof.

Example of the imidazolidinone compound which can be used is a compound represented by the following formula (7). 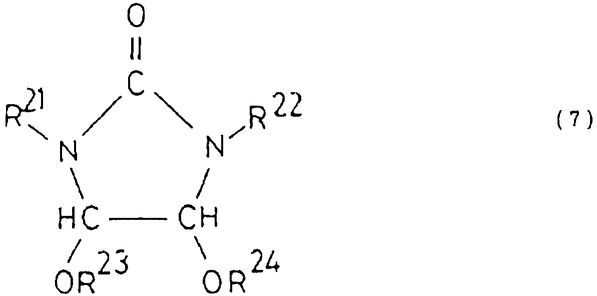 wherein R²¹ and R²² may be the same or different, and R²¹ and R²² each represents a hydrogen atom, a lower alkyl group, a lower acyl group, an alkoxy group or a -(CH₂-CH(CH₃)-O-)ᵣ-H group wherein r represents an integer of from 1 to 3, and R²³ and R²⁴ which may be the same or different each represent a hydrogen atom, a lower alkyl group, or a lower acyl group.

The lower alkyl group, lower alkoxy group, or lower acyl group in R²¹, R²², R²³, and R²⁴ in the above formula (7) means a straight chain or branched functional group having from 1 to 6 carbon atoms, and preferably from 1 to 4 carbon atoms. Examples of the lower alkyl group include methyl group, ethyl group n-propyl group, and isopropyl group. In particular, when R²¹ or R²² is a lower alkyl group, at least one hydrogen atom in the lower alkyl group can be replaced with hydroxyl group, cyano group, carboxyl group, or lower alkoxycarbonyl group. Specific examples of the substituents include ethoxycarbonyl and carbamoyl. Preferred examples of the substituted lower alkyl group include α-hydroxyethyl, β-hydroxyethyl, β-cyanoethyl, β-carbamoylethyl, β-carboxyethyl, and β-ethoxycarboxyethyl.

Specific examples of the imidazolidinone compound include:
4,5-dihydroxy-imidazolidinone,
1,3-dimethyl-4,5-dihydroxy-2-imidazolidinone,
1,3-diethyl-4,5-dihydroxy-2-imidazolidinone,
1,3-di(n-propyl)-4,5-dihydroxy-2-imidazolidinone,
1,3-di-isopropyl-4,5-dihydroxy-2-imidazolidinone,
1,3-di(α-dihydroxyethyl)-4,5-dihydroxy-2-imidazolidinone,
1,3-di(β-dihydroxyethyl)-4,5-dihydroxy-2-imidazolidinone,
1,3-dimethyl-4,5-dimethoxy-2-imidazolidinone,
1,3-dimethyl-4,5-diethoxy-2-imidazolidinone,
1,3-dimethyl-4,5-diisopropoxy-2-imidazolidinone,
1,3-dimethyl-4,5-diacetoxy-2-imidazolidinone,
1,3-di-(β-cyanoethyl)-4,5-dihydroxy-2-imidazolidinone,
1,3-di-(β-cyanoethyl)-4,5-dimethoxy-2-imidazolidinone,
1,3-di-(β-carbamoylethyl)-4,5-dihydroxy-2-imidazolidinone,
1,3-di-(β-carbamoylethyl)-4,5-dimethoxy-2-imidazolidinone,
1,3-di-(β-carboxyethyl)-4,5-dihydroxy-2-imidazolidinone,
1,3-di-(β-carboxyethyl)-4,5-dimethoxy-2-imidazolidinone,
1,3-di-(β-ethoxycarbonylethyl)-4,5-dihydroxy-2-imidazolidinone,
1,3-di-(β-ethoxycarbonylethyl)-4,5-dimethoxy-2-imidazolidinone.

Those imidazolidinone compounds can be used alone or as mixtures thereof.

The aldehyde compound having an aldehyde group is basically a compound represented by the following formula (9), and specific examples thereof include formaldehyde, glyoxal, acetaldehyde, a compound obtained by reacting a cyclic urea with glyoxal, acrylic aldehyde, and a compound obtained by reacting acrylic amide with glyoxal (acrylic amide-glyoxal reaction product copolymer). Those compounds can be used alone or as mixtures thereof.

R²⁶(CHO)ₓ (9)

wherein R²⁶ represents a hydrogen atom or a hydrocarbon group, having a molecular weight of from 14 to 1,000, and may contain oxygen atom, nitrogen atom or sulfur atom, and x is an integer of from 1 to 4, preferably 2 to 4 and R²⁶ may be deleted.

The acetal compound is obtained by addition reaction of a compound having an aldehyde group or a compound having a ketone group with an alcohol, and basically is a compound represented by the following formula (10). Specific examples of the acetal compound include, glycol acetal, and pentaerythritol bisacetal. Those compounds can be used alone or as a mixture thereof. 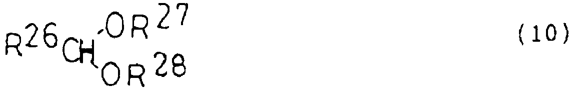 wherein R²⁶ represents a hydrogen atom or a hydrocarbon group, having a molecular weight of from 14 to 1,000, and may contain oxygen atom, nitrogen atom, or sulfur atom, and R²⁷ and R²⁸ each represents a lower alkyl group or a hydrocarbon group having a methylol group.

The active vinyl compound is basically a compound represented by the following formula (11). The compound has a double bond therein and has a high reactivity with cellulose. Examples of the active vinyl compound include methacrylic acid hydroxypropyltrimethylammoniumchloride, glyceroldimethacrylate, glycerol methacrylate acrylate, glycerol methacrylate alkenylate, diethylene glycol dimethacrylate, trimethylolpropane trimethacrylate, and zinc diacrylate. Those compounds can be used alone or as mixtures thereof. 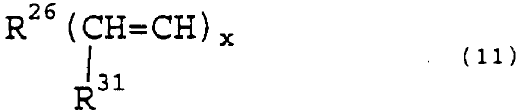 wherein R²⁶ represents a hydrocarbon group, having a molecular weight of from 14 to 1,000, and may contain oxygen atom, nitrogen atom or sulfur atom, and x is an integer of from 1 to 4 and R²⁶ may be deleted; R³¹ represents a hydrogen atom or a methyl group.

The aziridinyl compound is basically a compound represented by the following formula (12), and examples thereof include diphenyl-methane-bis-4,4'-N,N-diethylene urea and 2,2,4,4,6,6-hexa-(1-aziridinyl)-2,4,6-triphospha-1,3,5-triazine.

Those compounds can be used alone or as mixtures thereof. 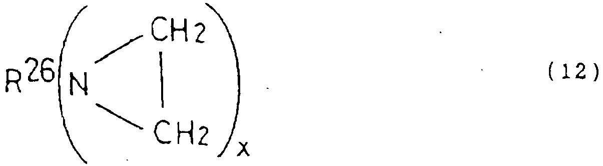 wherein R²⁶ represents a hydrocarbon group, having a molecular weight of from 14 to 1,000, and may contain oxygen atom, nitrogen atom or sulfur atom, and x is an integer of from 2 to 4.

The compound having a carboxyl group is basically a compound represented by the following formula (13), and is preferably a poly-carboxylic acid. Examples of the compound include 1,1,4,4-butane-tetracarboxylic acid, 1,2,3-tricarboxy-2, hydroxy-propane, bis-carboxymethyl ether, and 1,2-bis-carboxyhydroxy ethane. Those compounds can be used alone or as mixtures thereof.

R²⁶(COOH)ₓ (13)

wherein R²⁶ represents a hydrocarbon group, having a molecular weight of from 14 to 1,000, and may contain oxygen atom, nitrogen atom or sulfur atom, and x is an integer of from 2 to 4 and R²⁶ may be deleted.

The compound having an acyl group is basically a compound containing chlorine represented by the following formula (14), and examples thereof include stearic acid chloride, octadecylchloro-carbonic acid ester, and the like. Those compounds can be used alone or as mixtures thereof.

R²⁶(COCl)ₓ (14)

wherein R²⁶ represents a hydrocarbon group, having a molecular weight of from 14 to 1,000, and may contain oxygen atom, nitrogen atom or sulfur atom, and x is an integer of from 2 to 4.

The quaternary ammonium compound is basically a compound represented by the following formula (15), and the examples thereof include ethylene glycol bismethylpyridium chloride ether and the like. 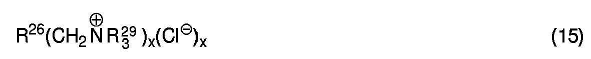 wherein R²⁶ represents a hydrocarbon group, having a molecular weight of from 14 to 1,000, and may contain oxygen atom, nitrogen atom or sulfur atom, R²⁹ represents a hydrogen atom or a lower alkyl group, and x is an integer of from 2 to 4 and R²⁶ may be deleted.

The amidophosphazene compound is a compound represented by the following formula (8), and examples thereof include an aminodiethylamidophosphazeneoligomer, atetraamino-n-propoxycyclotriphosphazene, pentaaminomonophenoxycyclotriphosphazene, and the like. Those compounds can be used alone or as mixtures thereof. $- {\left(N=P\right)}_{a} ⁢ {\left({NH}_{2}\right)}_{b} ⁢ {R}_{c}^{25}$
wherein R²⁵ represents a lower alkoxy group which may have a substituent, a phenoxy group which may have a substituent, a mono-lower alkylamino group, or a di-lower alkylamino group, a is an integer of 3 or more, and c is an integer of 1 or more, with proviso that b + c=2a and b/c≥1.

The compound having an isocyanate group is a compound represented by the following formula (16), Compounds represented by the formulae (17), (18), and (19) described below are examples of precursors of such compounds; they are heat-treated when bridging cellulose so that isocyanate compounds can be dissociated. Those compounds can be used alone or as mixtures thereof.

R²⁶(NCO)ₓ (16)

wherein R²⁶ represents a hydrocarbon group, having a molecular weight of from 14 to 1,000, and may contain oxygen atom, nitrogen atom and sulfur atom, and x is an integer of from 2 to 4. 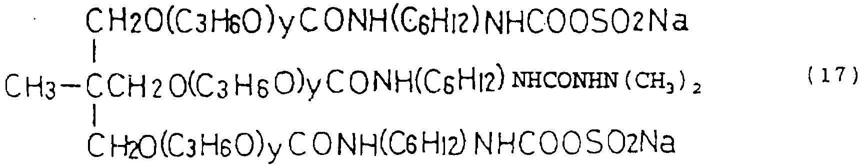 wherein y is an integer of from 2 to 4. 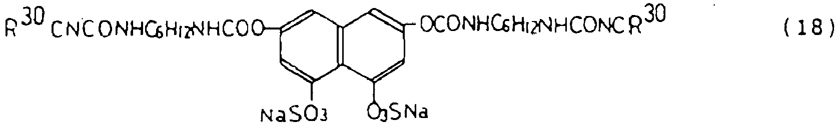 wherein R³⁰ represents a hydrogen atom, an alkyl group, an alkylene group, or a sulfone group. 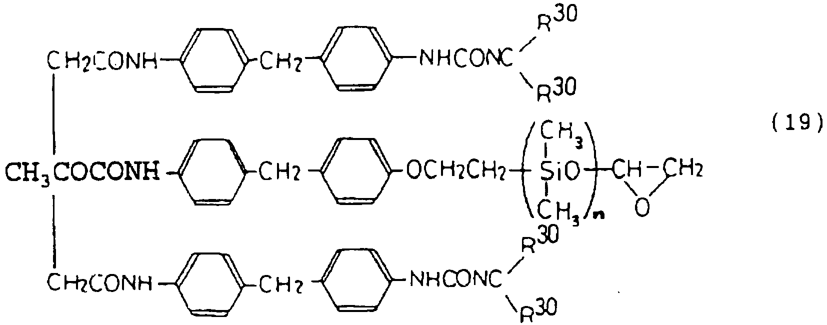 wherein R³⁰ represents a hydrogen atom, an alkyl group, an alkylene group, or a sulfone group and n is an arbitrary integer, preferably n is an integer from 1 to 200.

At least one member selected from the above-described epoxy compound, N-methylol compound, imidazolidinone compound, aldehyde compound having an aldehyde group, acetal compound, active vinyl compound, aziridinyl compound, compound having a carboxyl group, compound having an acyl group, quaternary ammonium compound, amidophosphazene compound, and compound having isocyanate group is reacted with cellulose in an amount of from 3 to 60% by weight, preferably from 15 to 40% by weight, and more preferably from 30 to 40% by weight, based on the weight of cellulose. If the amount of the compound reacted is less than 3% by weight, degradation of cellulose with microorganisms is accelerated and the carrier cannot be used over a long period of time. On the other hand, if the amount of the compound reacted exceeds 60% by weight, the inherent properties of cellulose are lost, and the carrier is liable to disintegrate, leading to deterioration of strength of the carrier. For those reasons, the compound should be used in an amount of the above-described range.

At least one member selected from the above-described epoxy compound having an epoxy group, N-methylol compound, imidazolidinone compound, aldehyde compound having an aldehyde group, acetal compound, active vinyl compound, aziridinyl compound, compound having a carboxyl group, compound having an acyl group, quaternary ammonium compound, amidophosphazene compound ,and compound having an isocyanate group is reacted with cellulose in such a manner that the cellulose is immersed in a solution prepared by dissolving the above-described compound in a solvent or a dispersion prepared by dispersing the above-described compound in a solvent and react ion is conducted at room temperature or under heating. The solvent which can be used is a polar solvent such as water, alcohols, ketones, or ethers. Those solvents can be used alone or as mixtures thereof. Preferred examples of the solvent include water, ethanol, methanol, isopropyl alcohol, dimethylsulfoxide, N,N-dimethyl-formamide, and the like. Reaction is conducted using those solvents at a temperature of from 10 to 200°C when humidity is 10 to 60% RH, and at a temperature of from 50 to 140°C when humidity is 60 to 100% RH, respectively. The reaction time can be controlled by, in particular, the shape of the cellulose. The reaction time is preferably from about 0.5 to 200 minutes, and more preferably from 30 to 60 minutes. If desired and necessary, the reaction product can be aged by allowing it to leave at room temperature in order to complete the reaction between the epoxy compound and the cellulose.

The reaction between the above-described compound and the cellulose can be conducted in the presence of a catalyst. Examples of the catalyst which can be used include an inorganic acid and an organic acid. Specific examples of the inorganic acid which can be used include hydrochloric acid (HCl), nitric acid (HNO₃), and the like. Examples of the organic acid which can be used include acetic acid (CH₃COOH), glycolic acid, oxalic acid and the like. Further examples of the catalyst which can be used include magnesium chloride (MgCl₂), zinc chloride (ZnCl₂), zinc nitrate (ZnNO₃), zinc borofluoride (Zn(BF₄)₂), magnesium borofluoride (Mg(BF₄)₂), ammonium chloride (NH₄Cl), alkanol amine, 2-methyl-2-aminopropanol hydrochloride, secondary ammonium phosphite, ammonium rhodanate, and the like.

The amount of the catalyst to be added is preferably from 3 to 12% by weight based on the weight of the above-described compound. The catalyst can previously be added to the solvent or can be added to the solution or the dispersion when cellulose is immersed therein.

Further, a method can be employed that at least one member selected from the epoxy compound having an epoxy group, the N-methylol compound, the aldehyde compound having an aldehyde group, the acetal compound, the active vinyl compound, the aziridinyl compound, the compound having a carboxy) group, the compound having an acyl group, the quaternary ammonium compound, the amidophosphazene compound, and the compound having an isocyanate group is added to a viscose liquid having dissolved therein cellulose in an amount of the above-described range, a pore-forming material is added thereto, and the resulting mixture is introduced into an appropriate mold and coagulated therein under heating, thereby obtaining a foamed porous cellulose derivative

According to another embodiment of the present invention, the carrier for immobilizing microorganisms comprises a porous cellulose obtained by coating cellulose with a compound which is obtained by reacting an epoxy compound having an epoxy group with a polyamine compound. By coating the cellulose with such a compound, chains of the cellulose are protected from enzymes which degrade cellulose, possessed by microorganisms. The compound obtained by reacting the epoxy compound having an epoxy group with the polyamine compound is coated on the cellulose in an amount of from 10 to 60% by weight, preferably from 15 to 50% by weight, and more preferably from 20 to 35% by weight, based on the weight of the porous cellulose. The polyamine compound is reacted with the epoxy compound in an amount of from 20 to 150% by weight, preferably from 30 to 120% by weight, and more preferably from 80 to 120% by weight, based on the weight of the epoxy compound. If the amount of the compound obtained by reacting the epoxy compound with the polyamine compound and coated on the cellulose is less than 10% by weight, the degradation of the cellulose with microorganisms is accelerated, and as a result, the carrier cannot be used over a long period of time. On the other hand, if the amount of the compound coated is more than 60% by weight, the specific gravity of the porous cellulose coated increases and a flowability of the carrier in a liquid treatment tank comes poor. As a result, it is difficult to increase the treatment capacity. For those reasons, it is desirable for the compound to be coated in an amount of the above-described range.

The above-described porous cellulose derivative can be used as the cellulose to be coated. For example, the porous cellulose derivative is obtained by reacting at least one member selected from an epoxy compound having an epoxy group, an N-methylol compound, an imidazolidinone compound, an aldehyde compound having an aldehyde group, an acetal compound, an active vinyl compound, an aziridinyl compound, a compound having a carboxyl group, a compound having an acyl group, a quaternary ammonium compound, an amidophosphazene compound, and a compound having an isocyanate group with cellulose.

Examples of the polyamine compound include a polyethyleneimine, a polyallylamine, a polyvinylamine, and the like.

The porous cellulose derivative or porous cellulose reacted or coated can prevent degradation with microorganisms and therefore can be used over a long period of time when it is used in a liquid treatment tank such as a nitrification tank or a denitrification tank.

The cellulose which can be used in the present invention is preferably a porous cellulose and more preferably a foamed cellulose. The shape of the cellulose can be a spherical shape, a cylindrical shape, a hollow cylindrical shape, or a dice shape. The foamed cellulose is obtained by chemically treating pulps having a high purity to convert the pulps into viscose which can be dissolved in an organic solvent, adding a pore-forming material thereto, introducing the resulting mixture into an appropriate mold, and heating to coagulate the same. The pores can be open cells or closed cells depending on the type of the treating liquid. The open cell means the structure that the pores present in the foamed cellulose are continuously connected, and the closed cell means the structure that the each pore is surrounded by septa. It is more preferable to use the foamed cellulose having open cells. The cellulose which is preferably used is one having a pore diameter, which is the standard of the size of the pore, of from 30 to 2,000 µm, preferably from 50 to 2,000 µm, and more preferably from 50 to 300 µm. The pore diameter can be measured by various methods, and in any measurement methods, cellulose having a pore diameter of from 30 to 2,000 µm can be used in the present invention.

The method of converting nitrogen compounds in a liquid using the carrier for immobilizing microorganisms, comprising the porous cellulose derivative or the porous cellulose according to the present invention includes the, following embodiments:

A method of converting nitrogen compounds in a liquid, which comprises: introducing the carrier for immobilizing microorganisms together with a liquid to be treated into a nitrification treatment tank under aerobic conditions, a denitrification treatment tank under anaerobic conditions, or a liquid treatment tank; and stirring the resulting mixture, thereby converting nitrogen compounds in the liquid to be treated;

A method of converting nitrogen compounds in a liquid, which comprises: introducing the carrier for immobilizing microorganisms together with a liquid to be treated into a nitrification treatment tank under aerobic conditions and a denitrification tank under anaerobic conditions; stirring the resulting mixture; and converting the nitrogen compounds in the liquid to be treated while flowing the entire amount of the liquid to be treated from the nitrification treatment tank under aerobic conditions to the denitrification treatment tank under anaerobic conditions; and

A method of converting nitrogen compounds in a liquid, which comprises: introducing the carrier for immobilizing microorganisms together with a liquid to be treated into a nitrification tank under aerobic conditions and a denitrification treatment tank under anaerobic conditions; stirring the resulting mixture; flowing the entire amount of the liquid to be treated from the nitrification treatment tank under aerobic conditions into the denitrification treatment tank under anaerobic conditions; and converting the nitrogen compounds in the liquid to be treated while circulating part of the liquid to be treated between the nitrification treatment tank under aerobic conditions and the denitrification treatment tank under anaerobic conditions.

The liquid to be treated according to the present invention is a liquid which contains nitrogen compounds and is intended to convert the nitrogen compounds into other nitrogen compounds. Examples of the liquid to be treated include groundwater, wastewater, sewage, and the like.

The nitrogen compounds contained in the liquid to be treated according to the present invention are compounds containing a nitrogen atom, and examples thereof include ammonia, nitrate, nitrite, and the like which are mainly contained in the nitrification treatment tank and the denitrification treatment tank. Further examples of the nitrogen compound include proteins, amino acids, and the like which are contained in an aeration tank used for BOD removal.

The liquid treatment tank means a treatment tank for treating a liquid which contains nitrogen compounds and is intended to convert the nitrogen compounds. Examples of the liquid treatment tank include a purification tank to be used in a sewage treatment plant or the like, an aeration tank to be used in a wastewater treatment, a nitrification tank, a denitrification tank, and the like.

The nitrification treatment tank, which is particularly used for a wastewater treatment, is a liquid treatment tank that can convert organic nitrogen into ammonia nitrogen, and further into nitrate nitrogen or nitrite nitrogen, by the action of nitrate oxidizing bacteria or nitrite oxidizing bacteria under aerobic conditions. In general, microorganisms that perform the nitrification treatment already exist in the liquid treatment tank. When a nitrification treatment tank is prepared by preventing from supplying carbon sources which are the nutrient for microorganisms, selection of microorganisms occurs. As a result, microorganisms such as nitrate oxidizing bacteria or nitrite oxidizing bacteria that can perform nitrification treatment are largely alive. Where the carrier for immobilizing microorganisms, which comprises the porous cellulose derivative or the porous cellulose according to the present invention is introduced into the tank during the above process, the microorganisms that perform nitrification treatment are immobilized onto the carrier and the capacity of the nitrification treatment can markedly be increased compared that of the conventional one. Genus Nitrosomonas as one example of the nitrite oxidizing bacteria and genus Nitrobacter as one example of the nitrate oxidizing bacteria, which perform the nitrification treatment, play an important role, respectively, in the nitrification tank.

The denitrification treatment tank is used in combination with the nitrification tank for the waste water treatment, or is used in the treatment of groundwater containing a large amount of nitrate, and the treatment is particularly conducted under anaerobic conditions. To the effect, the porous cellulose derivative or the porous cellulose of the present invention is introduced into the denitrification treatment tank to immobilize denitrifying bacteria in a similar manner as in the nitrification treatment tank, and nitrate is converted into nitrogen gas. Thus, nitrogen compounds are removed from the liquid to be treated. Examples of the denitrifying bacteria include genus Pseudomonas, genus Micrococcus, genus Spirillum, Achromobacter, genus Alcaligenes, genus Hydrogenomonas, genus Tiobacillus, and the like.

A hydrogen donor can be introduced into the denitrification tank. Examples of the hydrogen donor which can be used include methanol, acetic acid, ethanol, acetone, glucose, methyl ethyl ketone, isopropyl alcohol, and the like. The hydrogen donor functions to supply hydrogen in order to convert nitrate nitrogen or nitrite nitrogen into nitrogen gas (N₂) and water (H₂O).

According to the present invention, nitrogen compounds can be removed by treating a liquid to be treated in the treatment tank in the order of the nitrification treatment tank and the denitrification treatment tank. That is, the liquid containing nitrite nitrogen or nitrate nitrogen, which has been treated in the above-described nitrification treatment tank, is introduced into the denitrification treatment tank to convert nitrite nitrogen or nitrate nitrogen into nitrogen gas to remove nitrogen compounds from the liquid to be treated. Alternatively, a settling basin may be disposed between the nitrification treatment tank and the denitrification treatment tank. The liquid to be treated, which is flown from the nitrification treatment tank, is introduced into the settling basin, and succeedingly introduced into the denitrification treatment tank to remove nitrogen compounds in the liquid to be treated. Further, according to the invention, an aeration tank may be disposed before the nitrification treatment tank so as to introduce the liquid to be treated into the aeration tank to decrease the biochemical oxygen demand (BOD) in the liquid. The liquid thus treated is introduced into the settling basin, and thereafter, introduced into the nitrification treatment tank therefrom, to thereby remove nitrogen compounds in the liquid to be treated. Furthermore, part of the liquid, which has been treated in the treatment tank in the order of the nitrification treatment tank and the denitrification, can be circulated into the denitrification tank to further be treated therein. Moreover, nitrogen compounds contained in the liquid to be treated can be removed by disposing the settling basin between the denitrification tank and the nitrification tank and treating the liquid therein.

The liquid treatment tanks may contain the porous cellulose derivative or the porous cellulose in an amount of from 3 to 100 vol % based on the volume of the treatment tanks, the amount of which varies depending on the shape of the treatment tank. It is desirable for the nitrification treatment tank to contain the porous cellulose derivative or the porous cellulose in an amount of from 3 to 30 vol %, preferably from 5 to 25 vol %, and more preferably from 10 to 20 vol %, based on the volume of the nitrification treatment tank. That is, if the amount of the porous cellulose derivative or the porous cellulose is less than 3 vol%, the treatment capacity decreases. On the other hand, if the amount thereof exceeds 100 vol %, the flowability of the liquid is markedly impaired, and the treatment efficiency is markedly decreased due to the excess introduction. For the above reasons, it is preferred to use the porous cellulose derivative or the porous cellulose in the amount of the above-described range. Further, in the denitrification treatment tank, the carrier can be used by filling as in a column. In this case, the amount of the carrier which can be filled in the tank is from 50 to 100 vol % based on the volume of the treatment tank.

According to the carrier for immobilizing microorganisms, comprising the porous cellulose derivative or the porous cellulose, and to the conversion method of nitrogen compounds in a liquid using the carrier for immobilizing microorganisms, one or more members selected from an epoxy compound having an epoxy group, an N-methylol compound, an imidazolidinone compound, an aldehyde compound having an aldehyde group, an acetal compound, an active vinyl compound, an aziridinyl compound, a compound having a carboxyl group, a compound having an acyl group, a compound having an isocyanate group, a quaternary ammonium compound, and amidophosphazene compound crosslink the chains of glucose, thereby reducing or preventing disintegration of cellulose in a short period of time due to enzymes which decompose cellulose, and being possessed by microorganisms.

Further, by coating the cellulose with the compound obtained by reacting the epoxy compound with the polyamine compound, the cellulose is protected, thereby making it difficult to contact microorganisms with enzymes that decompose cellulose, and as a result, decomposition of the cellulose can be prevented.

When the carrier for immobilizing microorganisms, comprising the porous cellulose derivative is immersed into the liquid treatment tank such as the nitrification treatment tank or the denitrification treatment tank together with the liquid to be treated, and the resulting mixture is stirred therein, a large amount of the microorganisms are immobilized on the carrier due to the inherent characteristics of the cellulose with the result that the activity of microorganisms can be maintained.

Further, when the carrier for immobilizing microorganisms, comprising the porous cellulose is introduced into the liquid treatment tank such as the nitrification treatment tank or the denitrification treatment tank together with the liquid to be treated, and the resulting mixture is stirred therein, cationic charges possessed by the compound obtained by reacting the epoxy compound with the polyamine compound immobilize a large amount of microorganisms on the carrier with the result that the density of the microorganisms can be increased.

The present invention is explained in more detail with reference to the following specific embodiments.

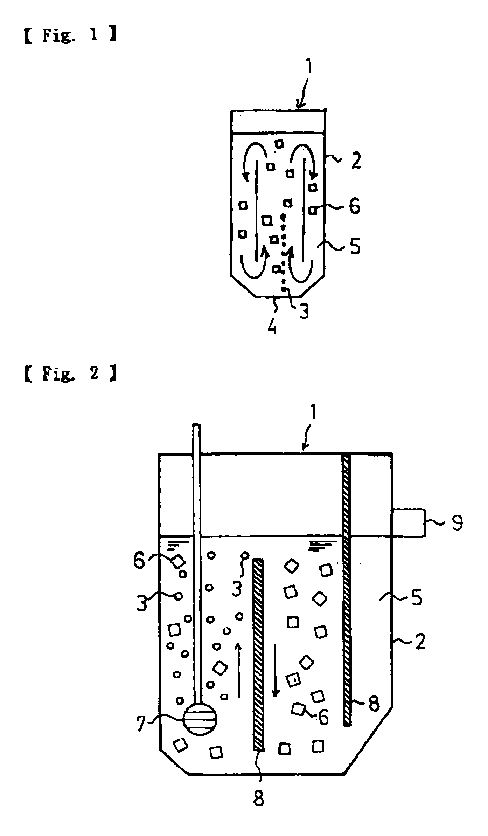

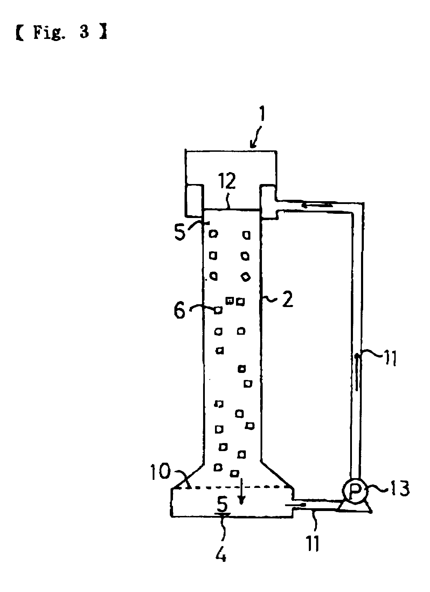

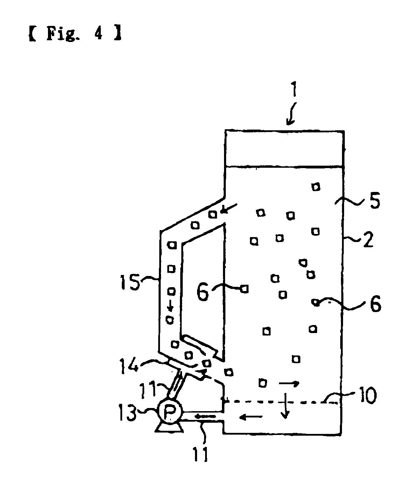

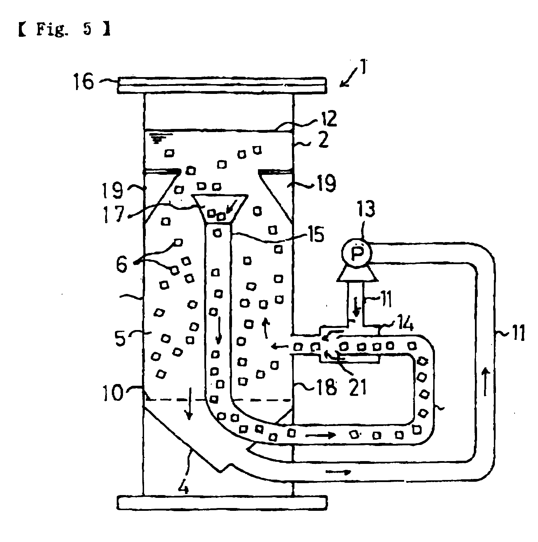

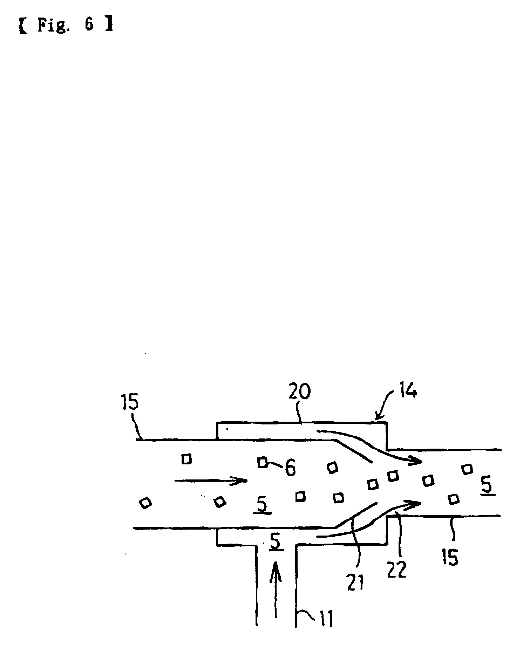

Specific examples of a liquid treatment tank which can be used in the present invention include the conventional liquid treatment tanks as shown in Figs. 1 to 4, and the liquid treatment tank as shown in Fig. 5. Figs. 1 and 2 exemplify the liquid treatment tanks that can stir a carrier for immobilizing microorganisms (hereinafter simply referred to as a "carrier") and a liquid to be treated by introducing a gas thereinto. Figs. 3, 4 and 5 show the liquid treatment tank where a stirring of the liquid to be treated and the carrier in the tanks is performed by circulating the liquid in the tank. In particular, the liquid treatment tanks shown in Figs. 4 and 5 include a liquid current jet device as shown in Fig. 6, which can stir the liquid to be treated together with the carrier.

The detailed description of the respective liquid treatment tanks will be made hereinafter.
Fig. 1 shows a tower-like gas lift-type liquid treatment tank 1. In this embodiment a stirring action due to an up-flow and a down-flow, indicated by the arrows in the figure, occurs inside of a tank body 2 by introducing a gas 3 such as nitrogen gas, air or the like from a bottom 4 of the liquid treatment tank 1, and a liquid 5 to be treated and a carrier 6 can be stirred with the result that the treatment is completed. In the tank shown in Fig. 1, a stirring means such as propellers can be employed in the thank body 2 to stir the liquid 5 to be treated.
Fig. 2 shows a box-like gas lift type liquid treatment tank 1. This liquid treatment tank 1 is provided with a gas supply means 7 which supplies a gas 3 from an air compressor or a nitrogen bomb into the liquid 5 to be treated, to treat the liquid under aerobic conditions or anaerobic conditions by introducing a gas 3 such as air into the liquid 5 to be treated. The liquid to be treated can be withdrawn from an outlet 9 after being treated through a partition wall 8. In this case, a stirring action occurs by an upflow and a down-flow as indicated by the arrows in the same manner as in the tank shown in Fig. 1, and the treatment can be completed.
Fig. 3 shows a down-flow expand-type liquid treatment tank 1. The tank is mainly used under anaerobic conditions, and a stirring is conducted by downwardly flowing the liquid 5 to be treated. The liquid treatment tank 1 has a screen 10 which partitions the inside thereof into two parts at the lower portion. The screen 10 separates the carrier 6 introduced together with the liquid 5 to be treated from the liquid 5 to thereby prevent the carrier 6 from entering into a reflux pipe 11 connected to the lower portion below the screen 10. Thus the liquid treatment can be completed. The reflux pipe 11 connected from a bottom 4 of the tank is connected to the tank body 2 at a position upper than a liquid level 12 of the liquid 5 to be treated in the tank body 2 via an outside of the tank. The liquid 5 to be treated is circulated through a pump 13 to perform a stirring.
Fig.4 shows an ejector type liquid treatment tank 1. The liquid treatment tank is partitioned into an upper portion and a lower portion by a screen 10. The lower portion below the screen 10 has a reflux pipe 11 which circulates the liquid 5 to be treated by a pump 13. A carrier recovery pipe 15 having a liquid current jet device 14 which joins with the reflux pipe 11 is arranged at the outside of the tank. The liquid 5 to be treated forcibly flows in the reflux pipe 11 by the pump 13, and flows into the liquid current jet device 14 to form a liquid current jet. The liquid 5 to be treated and the carrier 6 which circulate in the carrier recovery pipe 15 causes a driving force in the tank body 2 by the liquid current jet, thereby performing a fluidized stirring.
Fig. 5 shows another embodiment of the tank shown in Fig. 4, i.e, another example of the ejector type liquid treatment tank having a liquid current device 14. The liquid treatment tank 1 is mainly constituted of a carrier recovery pipe 15, a reflux pipe 11 having a circulating pump 13 in the middle thereof, and a liquid current jet device 14.

A tank body 2 is a cylindrical vessel having a bottom 4 and a lid 16, and is constituted so as to maintain an airtight property. The tank body 2 has a screen 10 which partitions the inside thereof into two portions. The screen 10 separates the carrier 6 introduced together with the liquid 5 to be treated from the liquid 5 so as to prevent the carrier 6 from entering into a reflux pipe 11. A carrier recovery pipe 15 equipped with a carrier recovery port 17 at the upper edge thereof is provided in the tank body 2. The carrier recovery pipe 15 sucks the carrier 6 and the liquid 5 to be treated at the position slightly below a liquid level 12 of the liquid 5 to be treated, and leads those to the outside of the tank body 2 through a tank wall 18. The carrier recovery pipe 15 is joined to the reflux pipe 11 in a liquid current jet device 14.

The reflux pipe 11 forcibly supplies the liquid 5 to be treated in the tank body 2 into the liquid current jet device 14 by a pump 13, and circulate the liquid 5 to be treated and the carrier 6 which flow in the carrier recovery pipe 15 which is joined to the liquid current jet device 14 by a driving force of the liquid current jet generated by the liquid current jet device 14. Further, in the inside of the tank body 2, slant walls 19 are provided around the periphery of the carrier recover port 17 on the entire periphery thereof or part of the periphery thereof. The carrier 6 floating toward the liquid level 12 can easily be collected around the periphery of the carrier recover port.

The theory of the liquid current jet device 14 used in Figs. 4 and 5 is explained using Fig. 6. A box 20 which receives the carrier recovery pipe 15 for recovering the carrier 6 is formed in the course of the reflux pipe 11. A leading-out edge 21 of the carrier recovery pipe 15 faces a narrow diameter portion 22 formed by narrowing the passage cross section in the box 20. When the liquid 5 to be treated, which was introduced into the box 20, passes through the narrow diameter portion 22, the flow rate of the liquid is accelerated due to the decrease of the cross section. As a result, the periphery of the narrow diameter portion 22 is under a negative pressure, and the liquid 5 to be treated containing the carrier 6 forms a liquid current jet from the carrier recovery pipe 15 by sucking into this negative pressure state.

The liquid treatment tank 1 described above can particularly employ the tank body 2 having a high airtight property, and at the periphery of which a water jacket can also be provided to maintain the temperature of the liquid 5 to be treated constant. Further, if required and necessary, oxygen electrode and dissolved oxygen meter to measure the dissolved oxygen in the liquid 5 to be treated, pH electrode and pH meter to measure pH of the liquid 5 to be treated, supply port to supply hydrocarbon, inorganic compounds, etc., and a device to discharge gas 3, etc., generated from the liquid 5 to be treated can be provided on the upper portion 5 of the tank body 2 or the tank wall 18, depending on the type of the liquid 5 to be treated. In particular, carbon sources can employ methanol, and the like which supply hydrogen atom. The inorganic compounds which can be used are compounds which adjust the pH of the liquid 5 to be treated, such as sodium hydroxide or hydrochloric acid.

The nitrification treatment tank and the denitrification treatment tank used in the present invention are explained in detail below.

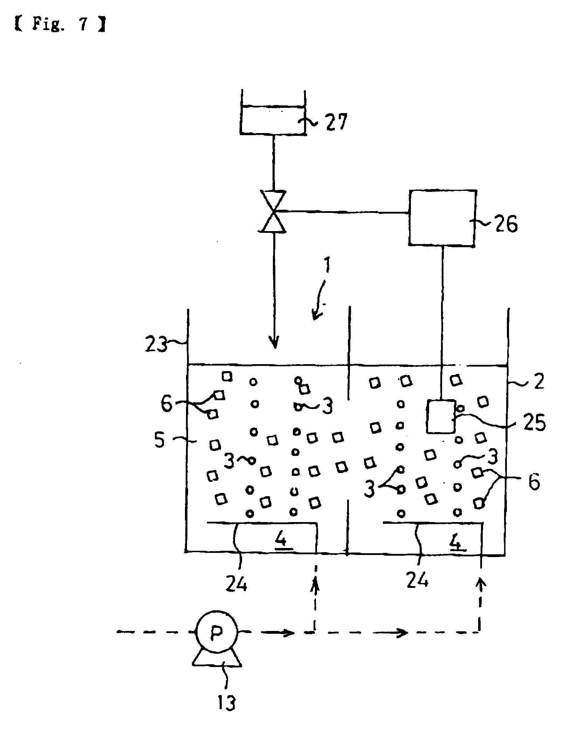

The nitrification treatment tank can employ the liquid treatment tank 1 shown in Figs. 1 to 5. When the treatment is conducted under aerobic conditions, it means the liquid treatment tank 1 where microorganisms which can perform the nitrification treatment (hereinafter referred to as "nitrifying bacteria") multiply, and nitrification treatment can be effected. The theory of this is explained by referring to Fig. 7. A blower 24 connected to the pump 13 which functions to stir and also supply an air is disposed at the bottom 4 of the nitrification treatment tank 23, and the tank contains the liquid 5 to be treated containing nitrifying bacteria and the carrier comprising the porous cellulose derivative or the porous cellulose of the present invention. By containing the carrier 6 in the tank, nitrifying bacteria contained in the liquid 5 to be treated are adhered or bonded to the carrier, and by the stirring action caused by feeding air into the liquid 5 to be treated with the blower 24, contact efficiency between the liquid 5 to be treated and the carrier 6 is increased, and the liquid 5 to be treated is nitrified. Further, in the case of nitrification, pH electrode 25 and pH meter 26 are disposed in the nitrification tank 23 in order to maintain pH constant, and while introducing an alkaline compound 27 such as sodium hydroxide as indicated by the arrow, the nitrification treatment can be conducted with preventing pH from being decreased, and growth environment of microorganisms can be maintained constant. Nitrogen compounds mainly comprising ammonia nitrogen (NH₄-N) present in the liquid 5 to be treated in the nitrification tank 23 are converted into nitrate nitrogen (NO₃-N) and nitrite nitrogen (NO₂-N).

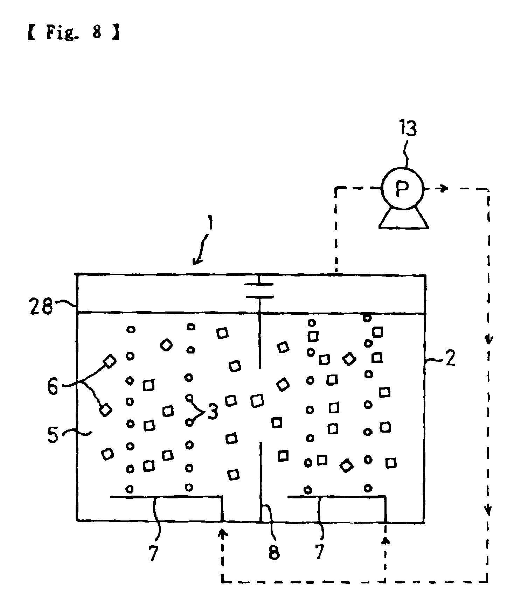

Similar to the above-described nitrification tank 23, the liquid treatment tank 1 shown in Figs. 1 to 5 can also be employed in the denitrification tank 28 shown in Fig. 8. The theory of the denitrification is explained by referring to Fig. 8. When the treatment is conducted in the liquid treatment tank 1 under anaerobic conditions, i.e., oxygen-free conditions, microorganisms which perform denitrification (hereinafter referred to as "denitrifying bacteria") contained in the liquid 5 to be treated multiply. Thus the denitrification tank 28 treats the liquid 5 to be treated. In particular, a tank that is closed and can prevent contamination of oxygen is suited to be used as the denitrification tank 28. The carrier 6 comprising the porous cellulose derivative or the porous cellulose, and the liquid 5 to be treated are contained in the denitrification tank 28, where denitrifying bacteria are adhered or bonded to the carrier 6, and nitrate nitrogen (NO₃-N) and nitrite nitrogen (NO₂-N) contained in the liquid 5 to be treated are converted into water and nitrogen gas. The nitrogen gas is separated from the denitrification tank 28 and released into the atmosphere with the result that nitrogen compounds are removed from the liquid to conduct denitrification. In this case, if desired and necessary, hydrocarbon such as ethanol, which is hydrogen donor, can be supplied to the liquid 5 to be treated. Further, the denitrification tank 28 can conduct a treatment while introducing oxygen-free gas such as nitrogen gas into the liquid 5 to be treated with a gas supply means 7 through the pump 13.

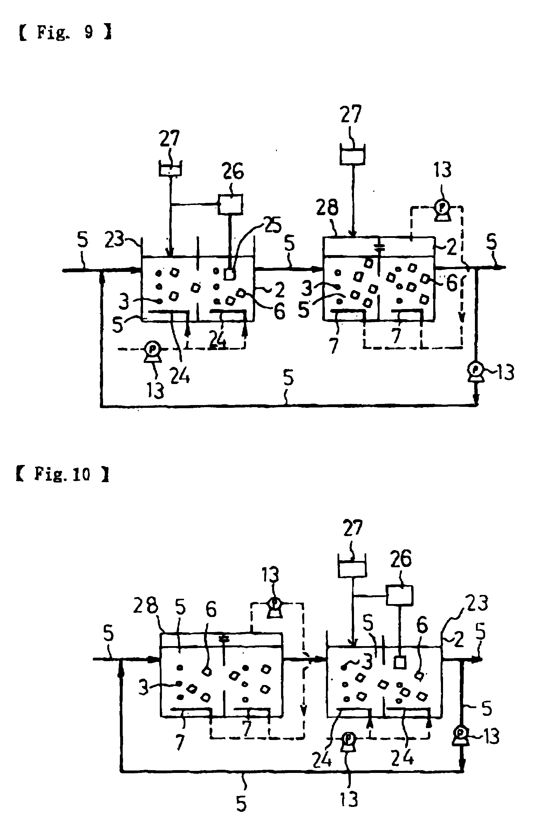

The nitrification tank 23 and the denitrification tank 28 can be combined for the treatment of the liquid 5 to be treated. This method is shown in Fig. 9. The nitrification tank 23 under aerobic conditions and the denitrification tank 28 under anaerobic conditions are disposed in this order. Each tank contains the liquid 5 to be treated and the carrier 6 comprising the porous cellulose derivative or the porous cellulose. In the nitrification tank 23, nitrifying bacteria are adsorbed or adhered onto the carrier 6 to immobilize the same on the carrier 6, and in the denitrification tank 28, denitrifying bacteria are adsorbed or adhered onto the carrier 6 to immobilize the same on the carrier 6. That is, thus treated nitrogen compounds in the liquid 5 to be treated in the nitrification tank 23 are converted from ammonia nitrogen (NH₄-N) to nitrate nitrogen (NO₃-N) and nitrite nitrogen (NO₂-N). The liquid 5 to be treated is transferred into the denitrification tank 28, and the nitrate nitrogen (NO₃-N) and the nitrite nitrogen (NO₂-N) are converted into nitrogen gas and water with the denitrifying bacteria while supplying the compound 27 such as hydrocarbon.

Fig 10. shows the configuration that the denitrification tank 28 and the nitrification tank 23 are disposed in this order reversely to the above-described Fig. 9, and each tank contains the liquid 5 to be treated and the carrier 6 comprising the porous cellulose derivative or the porous cellulose to conduct the treatment. In this case, the organic compounds such as hydrocarbon contained in the liquid 5 to be treated function as hydrogen donor. The nitrate nitrogen (NO₃-N) and nitrite nitrogen (NO₂-N) contained in the liquid 5 to be treated are converted into water and nitrogen gas with the denitrifying bacteria, and the nitrogen compounds are thus removed from the liquid. The thus-treated liquid 5 to be treated is transferred into the nitrification tank 23, and the ammonia nitrogen (NH₄-N) is converted into nitrate nitrogen (NO₃-N) and nitrite nitrogen (NO₂-N) in the same manner as described above. Part of the liquid 5 to be treated is returned to the denitrification tank 28 and the treatment is conducted in the same manner as described above Nitrogen compounds contained in the liquid 5 to be treated are further converted into nitrogen gas and water, and finally the nitrogen compounds are removed from the liquid.

The present invention is described in more detail with reference to the following examples showing that the carrier for immobilizing microorganisms is prepared and nitrogen compounds contained in the liquid are converted in the nitrification tank and the denitrification tank using the carrier. It should however be understood that the invention is not construed as being limited to the examples. Unless otherwise indicated, all percents, parts, ratios, and the like are by weight.

### EXAMPLES 1 TO 4

A carrier composed of a porous cellulose derivative was prepared using an epoxy resin compound in the following manner.

Four types of the carriers having different bonding amounts were prepared using 5 to 30% glycerol diglycidyl ether of an epoxy compound. 50 g of a mixed solution composed of glycerol diglycidyl ether in a concentration of 5 to 30%, ethanol aqueous solution in a concentration of 20%, and thorium in a concentration of 1% were prepared. 10 g of foamed products (a cube of 5 x 5 x 5 mm; pore size 100 µm) were immersed in the resulting mixed solution, and reaction was conducted in a reaction vessel at 121°C for 15 minutes. Unreacted sodium hydroxide, ethanol and glycerol diglycidyl ether were removed to obtain a carrier composed of a porous cellulose derivative. The bonding amount of the glycerol diglycidyl ether was measured by a method that the actual bonding amount was obtained by subtracting the amount of the porous cellulose derivative before reaction from the weight of the porous cellulose after reaction, and the bonding amount per 10 g of the cellulose was obtained. The carrier obtained in Example 1 had the bonding amount of about 0.3 g (about 3%), the carrier obtained in Example 2 had the bonding amount of about 0.7 g (about 7%), the carrier obtained in Example 3 had the bonding amount of about 1.8 g (about 18%), and the carrier obtained in Example 4 had the bonding amount of about 3.0 g (about 30%).

### EXAMPLE 5

A carrier composed of the porous cellulose derivative and having a bonding amount of about 1.7 g (about 17%) was obtained in the same manner as in Examples 1 to 4, except that cellulose foamed products (a cube of 5 x 5 x 5 mm) having a pore diameter of 50 µm were used.

### EXAMPLE 6

A carrier composed of the porous cellulose derivative and having a bonding amount of about 1.7 g (about 17%) was obtained in the same manner as in Examples 1 to 4, except that cellulose foamed products (a cube of 5 x 5 x 5 mm) having a pore diameter of 500 µm were used.

### EXAMPLE 7

A carrier composed of the porous cellulose derivative and having a bonding amount of about 1.7 g (about 17%) was obtained in the same manner as in Examples 1 to 4, except that cellulose foamed products (a cube of 5 x 5 x 5 mm) having a pore diameter of 1,260 µm were used.

### EXAMPLES 8 TO 11

Four types of the carriers having different bonding amounts were prepared using 1,3-dimethyl-4,5-dihydroxy-2-imidazolidinone. 10 g of cellulose foamed products (a cube of 10 x 10 x 10 mm; pore diameter 1,260 µm) were immersed in 50 g of a mixed solution of 1,3-dimethyl-4,5-dihydroxy-2-imidazolidinone in a concentration of 5 to 30%, and zinc borofluoride in a concentration of 0.3 to 3%. Reaction was conducted in the same manner as in Examples 1 to 4. The cellulose foamed products thus treated were pre-dried at 110 °C for 5 minutes, subjected to heat treatment at 130°C for 15 minutes, and washed with water to obtain a carrier composed of the porous cellulose derivative. The bonding amount of the 1,3-dimethyl-4,5-dihydroxy-2-imidazolidinone was measured by subtracting the amount of the porous cellulose derivative before reaction from the weight of the porous cellulose after reaction. The carrier obtained in Example 8 had a bonding amount of about 0.3 g (about 3%), the carrier obtained in Example 9 had a bonding amount of about 0.8 g (about 8%), the carrier obtained in Example 10 had a bonding amount of about 1.6 g (about 6%), and the carrier obtained in Example 11 had a bonding amount of about 2.8 g (about 28%).

### EXAMPLE 12

A carrier was obtained in the same manner as in Examples 8 to 11, except that a melamine-formaldehyde resin represented by the formula (20) which was an N-methylol compound and 2-methyl-2-aminopropanol hydrochloric acid salt as a catalyst were used. The bonding amount in the carrier was measured in the same manner as in Examples 8 to 11 and was found to be about 0.7 g (about 7%). 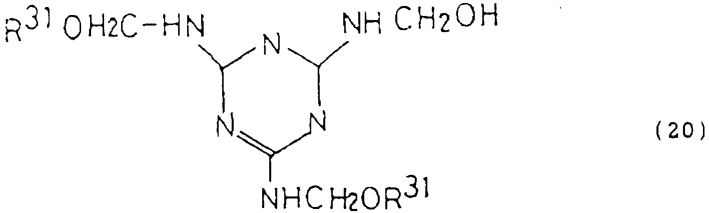 wherein R³¹ represents an alkyl group.

### EXAMPLE 13

A carrier was obtained in the same manner as in Examples 8 to 11, except that a cyclic urea-glyoxal reaction product represented by the formula (21) which was an aldehyde compound was used. The bonding amount in the carrier was measured in the same manner as in Examples 8 to 11, and was found to be about 1.5 g (about 15%). 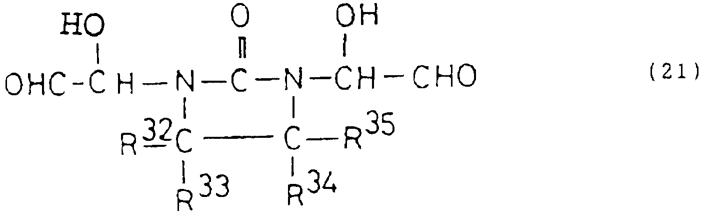 wherein R³², R³³, R³⁴ and R³⁵ each represents H, OH, OR³⁶ or COOR³⁶, wherein R³⁶ represents an alkyl group.

### EXAMPLE 14

A carrier was obtained in the same manner as in Examples 8 to 11, except that diphenylmethane-bis-4,4'-N,N-diethylene urea represented by the formula (22) which was an aziridinyl compound was used. The bonding amount in the carrier was measured in the same manner as in Examples 8 to 11, and was found to be about 1.2 g (about 12%).

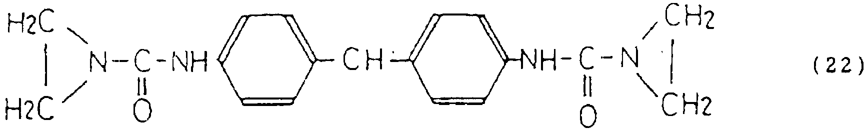

### EXAMPLE 15

A carrier composed of cellulose having coated thereon a compound obtained by reacting an epoxy compound with a polyamine compound was prepared in the following manner.

50 g of an aqueous solution composed of a polyethylene imine having a molecular weight of 70,000 in a concentration of 2.8%, and ethyleneglycol diglycidyl ether in a concentration of 3.3% were prepared. 10 g of cellulose foamed products (a cube of 5 x 5 x 5 mm; pore diameter 100 µm) were uniformly immersed in the aqueous solution, and allowed to stand for 2 hours. The products thus treated were dried in an oven at 80 to 90°C, and then heated in a thermostat at 120°C for 30 minutes. After completion of the reaction, the products were washed with tap water, dewatered, washed with ion-exchanged water, and then dried in a thermostat at 60°C to obtain a carrier. The amount of the reaction product coated was about 3.1 g (about 31%).

### [Biodegradation Inhibition Test]

Biodegradation inhibition test was conducted on the carriers obtained in Examples 1 to 4. The measurement method is as follows.

Cellulase (trade name: R10, a product of Yakult Co., Ltd.) was added to a citric acid-sodium phosphate buffer solution having pH of 4.5 such that the concentration thereof was 0.5%, thereby preparing a cellulase solution. The carrier obtained in Example 1 was added to the buffer solution. L-type test tube containing the resulting mixture was shaked at 72 revolutions per minute in a mono-type shaking apparatus, and time that was required for degradation of the cellulose was measured. The cellulase solution was exchanged with a fresh solution twice a week. Untreated cellulose foamed product was used as a control.

The results obtained are shown in Table 1 below.

The term "Biodegradation inhibition" as mentioned in Table 1 means the following estimation: $Biodegradion inhibition = Degradation time of Example / 4 hours ; therein 4 hours is the degradation time of a control .$

**TABLE 1**

| | Degradation time (hours) | Biodegradation Inhibition |
|---|---|---|
| Example 1 | 38 | 9 |
| Example 2 | 350 | 87 |
| Example 3 | 1000 or more | 250 or more |
| Example 4 | 2000 or more | 500 or more |
| Example 5 | 1000 or more | 250 or more |
| Example 6 | 1000 or more | 250 or more |
| Example 7 | 1000 or more | 250 or more |
| Example 8 | 40 | 10 |
| Example 9 | 165 | 40 |
| Example 10 | 300 | 75 |
| Example 11 | 800 | 200 |
| Example 12 | 75 | 19 |
| Example 13 | 200 | 50 |
| Example 14 | 120 | 30 |
| Example 15 | 1000 or more | 250 or more |
| Control | 4 | 1 |

It can be clearly understood from the data shown in Table 1 above that the carriers obtained in Examples 1 to 15 are resistant to cellulase and have a sufficient degradation inhibition.

### [Nitrification test of converting nitrogen compounds contained in a liquid in nitrification tank under aerobic conditions using a carrier comprising a porous cellulose derivative]

A box-like gas lift-type liquid treatment tank 1 shown in Fig. 2 was used as a nitrification tank. When ammonia nitrogen (NH₄-N) in a liquid was converted into other nitrogen compound using each of the carriers for immobilizing microorganisms obtained in Examples 3, 5, 6 and 7, residual amount of ammonia nitrogen was measured. The details thereof are described in Examples 16 to 19 below.

### EXAMPLE 16

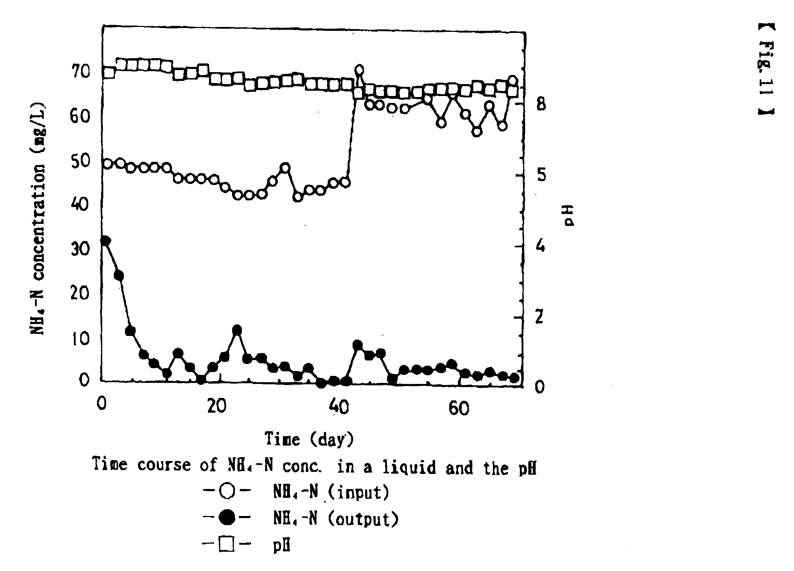

A 3 liter volume liquid treatment tank as shown in Fig. 2 was used as the nitrification treatment tank. An artificially synthesized inorganic waste water having the components shown in Table 2 below was prepared. Activated sludges were adsorbed and immobilized onto the porous cellulose derivative used in Example 3 to prepare The carrier. The artificially synthesized inorganic waste water prepared above was treated with the carrier in the nitrification tank for 70 days while adjusting the pH of the waste water at around 8.0. Concentrations of ammonia nitrogen (NH₄-N) introduced and ammonia nitrogen (NH₄-N) discharged were measured. The measurement was conducted under the conditions that the amount of dissolved oxygen was 4 mg/ml, the maximum treatment amount in one day was 36 liters, and the treatment amount per one hour was 1,200 ml. The carrier comprising the porous cellulose derivative was introduced into the nitrification tank in an amount of 10 vol % based on the volume of the nitrification tank, and the amount of ammonia nitrogen (NH₄-N) introduced was changed to 70 mg/l after 40 days. As a result, the treatment capacity of the carrier was 315 mg-N/l-carrier/hr. The results obtained are shown in Fig. 11

**TABLE 2**

| Components | Amount(mg/l) |
|---|---|
| NH₄Cl" | 158.2 |
| NaHPO₄ • 12H₂O | 23.1 |
| NaCl | 10.1 |
| KCl | 4.7 |
| CaCl₂ • 2H₂O | 4.7 |
| MgSO₄ • 7H₂O | 16.7 |
| NaHCO₃ | 937.2 |

| | |
|---|---|
| ": NH₄-N = 40 mg/l | |

### EXAMPLE 17

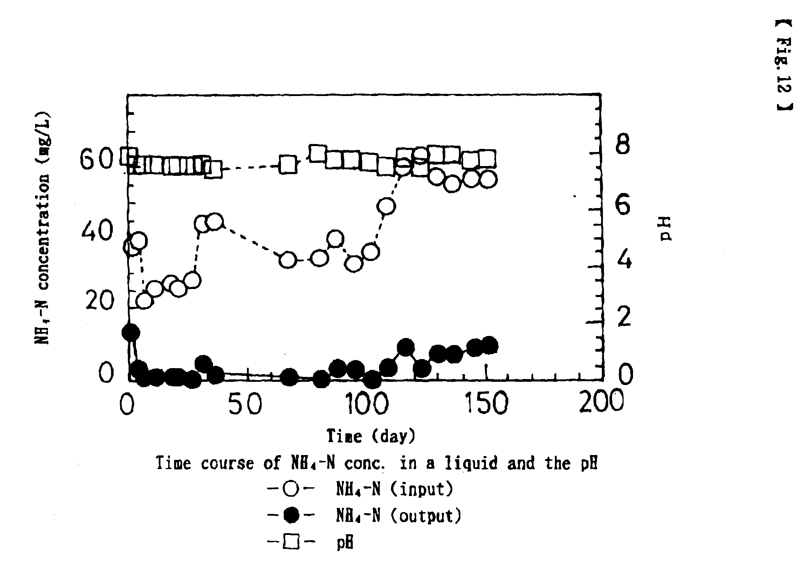

Nitrification test was conducted for 150 days in the same manner as in Example 15, except that the carrier obtained in Example 5 was used. The results obtained are shown in Fig. 12.

### EXAMPLE 18

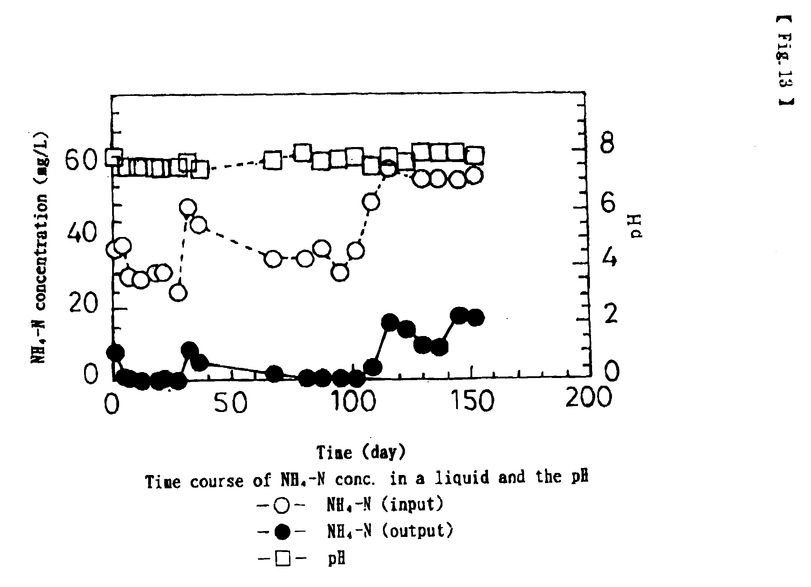

Nitrification test was conducted for 150 days in the same manner as in Example 15, except that the carrier obtained in Example 6 was used. The results obtained are shown in Fig. 13.

### EXAMPLE 19

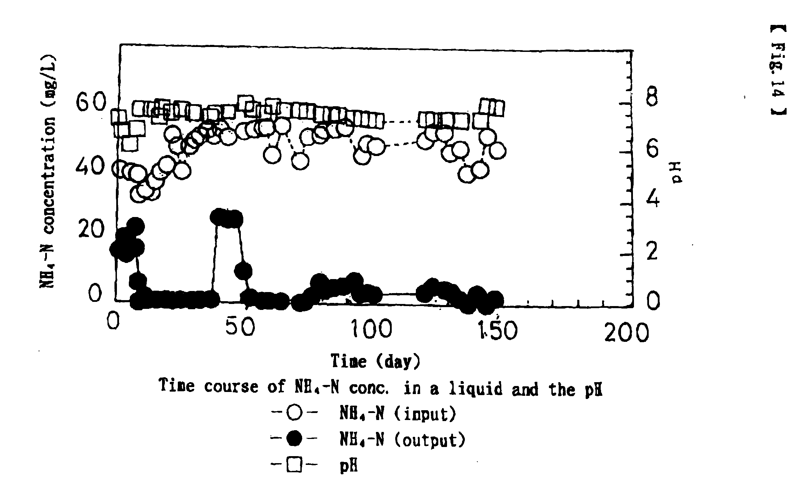

Nitrification test was conducted for 150 days in the same manner as in Example 15, except the carrier obtained in Example 7 was used. The results obtained are shown in Fig. 14.

It is apparent from Fig 11 that the ammonia nitrogen (NH₄-N) that was introduced could be converted with high efficiency. It is also apparent from Figs. 12, 13 and 14 that the ammonia nitrogen (NH₄-N) in the liquid can be converted with high efficiency even in the use of the carriers having different pore sizes.

### [Denitrification test of converting nitrogen compounds contained in a liquid in denitrification tank under aerobic conditions using carrier comprising the porous cellulose derivative]

Nitrate nitrogen (NO₃-N) which was nitrogen compound in a liquid was converted using the liquid treatment tanks as shown in Figs. 1, 3 and 5 as the denitrification tank, and the residual amount of nitrate nitrogen was measured. The details thereof are described by referring to Examples 20 to 23 described below.

### EXAMPLE 20

The tower-like gas lift type liquid treatment tank (volume 1.3 liters) as shown in Fig. 1 was used as the denitrification tank. Conversion of microorganisms in a liquid was conducted in the tank while adjusting the maximum treatment amount in one day was 2.8 liters, and the amount of acetic acid was 20 mM per mole of the carbon source in a liquid to be treated.

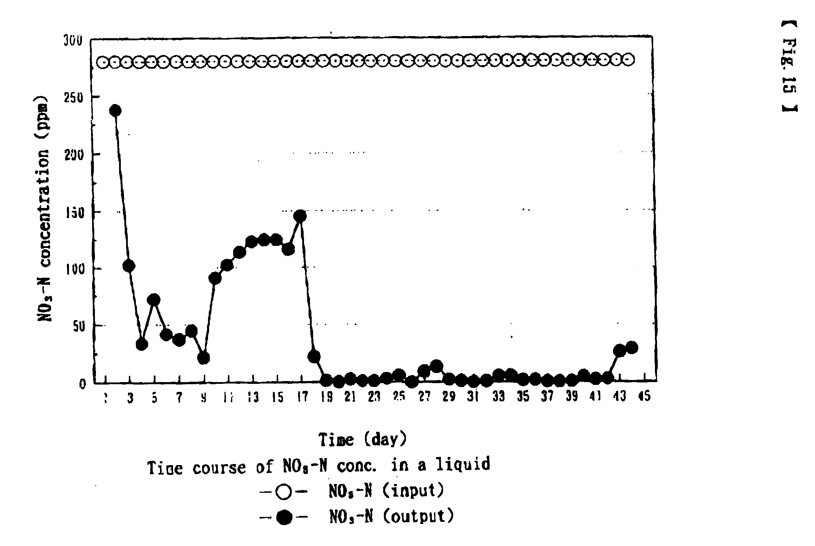

An artificially synthesized inorganic waste water having the components shown in Table 3 below was prepared as a liquid to be treated while supplying acetic acid such that the concentration of nitrate nitrogen (NO₃-N) was 280 ppm. Activated sludges were adsorbed and immobilized onto the porous cellulose derivative obtained in Example 12 to prepare the carrier. The artificially synthesized waste water prepared above was treated with the carrier in the denitrification tank for 45 days. The residual amount of nitrate nitrogen (NO₃-N) in the liquid to be treated was measured. The carrier comprising the porous cellulose derivative was introduced into the tank in an amount of 10 vol % based on the volume of the tank. The results obtained are shown in Fig. 15. The treatment capacity of the carrier was 56 mg-N/l-carrier/hr.

### EXAMPLE 21

The down-flow expand type liquid treatment tank (volume 0.6 liter) was used as the denitrification tank. Conversion of microorganisms in the liquid was conducted while adjusting the maximum treatment amount in one day was 1.3 liters, and the concentration of methanol was 40 mM per mole of the carbon source in the liquid to be treated.

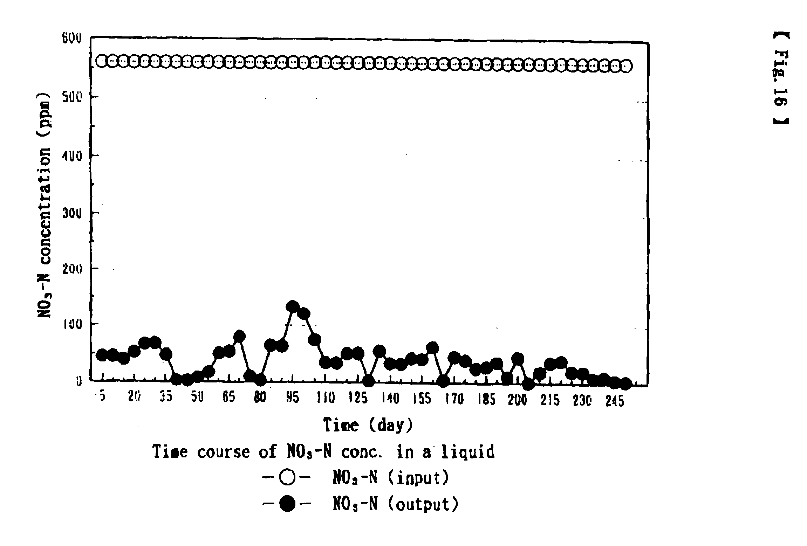

An artificially synthesized inorganic waste water having compositions shown in Table 3 below was prepared as the liquid to be treated while supplying nitric acid such that the concentration of nitrate nitrogen (NO₃-N) was 560 ppm. Activated sludges were adsorbed and immobilized onto the porous cellulose obtained in Example 12 to prepare the carrier. The artificially synthesized waste water prepared above was treated with the carrier in the tank for 245 days. The residual amount of nitrate nitrogen (NO₃-N) in the liquid was measured. The carrier comprising the porous cellulose derivative was introduced into the tank in an amount of 10 vol% based on the volume of the tank. The results obtained are shown in Fig. 16. The treatment capacity of the carrier was 508 mg-N/l- carrier/hr.

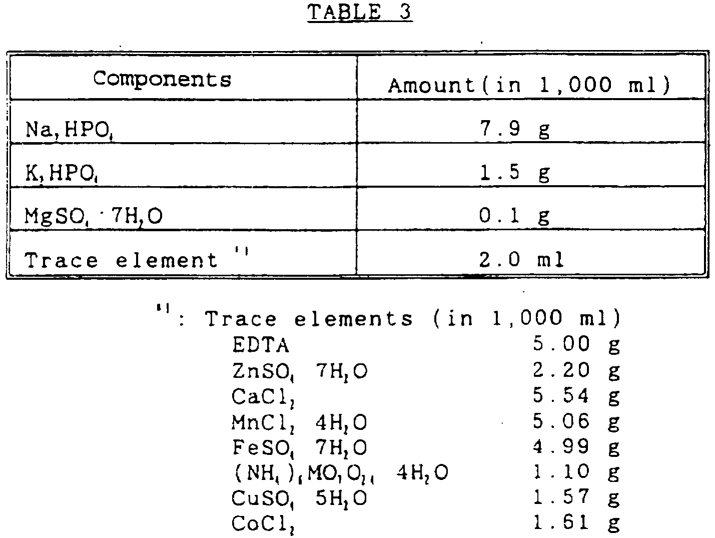

### EXAMPLE 22

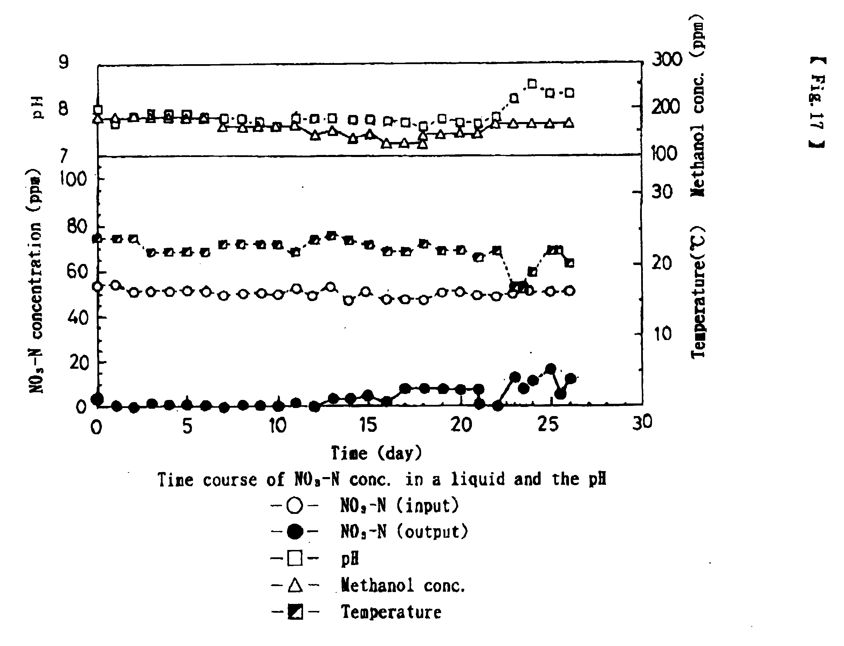

An ejector type liquid treatment tank (volume 15 liters) as shown in Fig. 5 was used as the denitrification tank. The maximum treatment amount was adjusted to 360 liters per day. Activated sludges were adsorbed and immobilized onto the porous cellulose obtained in Example 12 to prepare the carrier. Groundwater was used as a liquid to be treated. Denitrification treatment of groundwater was conducted with the carrier in the tank while supplying nitric acid such that the concentration of nitrate nitrogen (NO₃-N) in groundwater was 50 ppm. Further, methanol was introduced into the tank through an inlet for carbon source, and according to the need, hydrochloric acid was introduced into the tank through an inlet for inorganic compound in order to maintain the pH in the tank at 8.0. The treatment was conducted while circulating the liquid to be treated and the carrier with a flow current jet device. The carrier was introduced into the tank in an amount of 16 vol % based on the volume of the tank. The results obtained are shown in Fig. 17. The treatment capacity of the carrier was 312 mg-N/l-carrier/hr.

It is apparent from Figs. 15, 16 and 17 that nitrate nitrogen (NO₃-N) in the liquid could be converted with high efficiency.

According to the carrier for immobilizing microorganisms, comprising the porous cellulose derivative or the porous cellulose, and the method of converting nitrogen compounds contained in a liquid using the carrier of the present invention, at least one member selected from an epoxy compound having an epoxy group, an N-methylol compound, a carbonyl compound, an acetal compound, an active vinyl compound, an aziridinyl compound, an aldehyde compound having an aldehyde group, a compound having an acyl group, a quaternary ammonium compound, an amidophosphazene compound, and a compound having an isocyanate group crosslink the chain of glucose which constitutes cellulose, thereby reducing disintegration of the cellulose due to enzymes which degraded the cellulose, possessed by microorganisms. As a result, the carrier can be used over a long period of time.

Further, according to the carrier for immobilizing microorganisms, comprising the porous cellulose having coated thereon a compound obtained by reacting an epoxy compound with a polyamine compound, it makes it difficult for the carrier to contact microorganisms by protecting the cellulose with the coating of the compound, and makes it possible to prevent the cellulose from degradation. Thus, degradation of the cellulose can be controlled with the result that the carrier can be used over a long period of time. When the carrier for immobilizing microorganisms, comprising the porous cellulose derivative is introduced into the liquid treatment tank such as the nitrification treatment tank or the denitrification treatment tank together with the liquid to be treated, and the resulting mixture is stirred, a large amount of microorganisms are immobilized onto the porous cellulose derivative due to the inherent characteristics of the cellulose, and activity of the microorganisms can be maintained. As a result, the treatment capacity of the carrier can be increased.

Further, when the carrier for immobilizing microorganisms, comprising the porous cellulose is introduced into the liquid treatment tank such as the nitrification treatment tank or the denitrification treatment tank together with the liquid to be treated, and the resulting mixture is stirred, cationic charges possessed by the compound obtained by reacting an epoxy compound with a polyamine compound enables to immobilize a large amount of microorganisms onto the carrier, thereby increasing the density of the immobilized microorganisms. As a result, the treatment capacity of the carrier can be increased.

In addition, since cellulose which is a natural material is used, the carrier can be incinerated similar to papers, and even if the carrier is embedded in the earth, the cellulose disintegrates with the passage of time. Thus, the carrier after use can easily be disposed.

While the invention has been described in detail and with reference to specific examples thereof, it will be apparent to one skilled in the art that various changes and modifications can be made therein without departing from the spirit and scope thereof.

## Claims

1. A carrier for immobilizing microorganisms, comprising a porous cellulose derivative.

2. A carrier for immobilizing microorganisms according to claim 1, wherein the porous cellulose derivative is obtained by reacting at least one of an epoxy compound having an epoxy group, an N-methylol compound, an imidazolidinone compound, an aldehyde compound having an aldehyde group, an acetal compound, an active vinyl compound, an aziridinyl compound, a compound having a carboxyl group, a compound having an acyl group, a compound having an isocyanate group, a quaternary ammonium compound, and an amidophosphazene compound, with cellulose.

3. A carrier for immobilizing microorganisms, comprising a porous cellulose having coated thereon a compound obtained by reacting an epoxy compound having an epoxy group with a polyamine compound.

4. A carrier for immobilizing microorganisms as claimed in claim 3, wherein the polyamine compound is reacted with the epoxy compound in an amount of from 20 to 150% by weight based on the weight of the epoxy compound.

5. A carrier for immobilizing microorganisms as claimed in claim 3 or 4, wherein the compound obtained by reacting the epoxy compound with the polyamine compound is coated on the cellulose in an amount of from 10 to 60% by weight based on the weight of the cellulose.

6. A carrier for immobilizing microorganisms as claimed in any one of claims 2 to 5, wherein the epoxy compound is at least one member selected from the following formulae (2), (3), (4), (5) and (6): 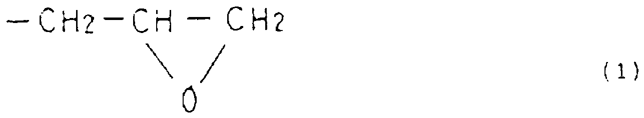 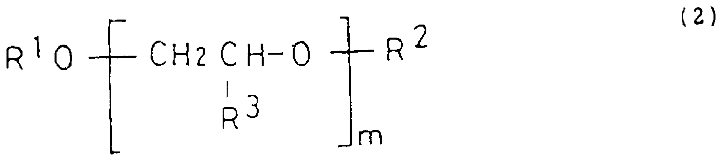 wherein m represents an integer of from 0 to 50; R¹ and R² may be the same or different and represent a hydrogen atom, a methyl group or a group of formula (1) above and R³ represents a hydrogen atom or a methyl group; with the proviso that at least one of R¹ and R² represents a group of formula (1); 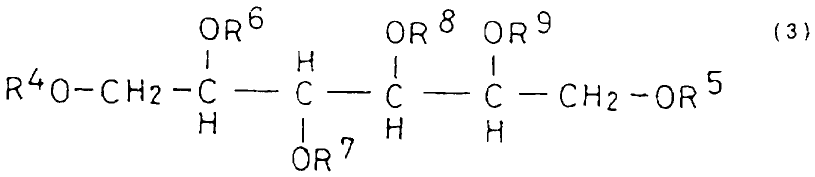 wherein R⁴, R⁵, R⁶, R⁷, R⁸ and R⁹ may be the same or different and represent a hydrogen atom, a phenyl group, a methyl group or a group of formula (1) above, with the proviso that at least one of R⁴, R⁵, R⁶, R⁷, R⁸ and R⁹ represents a group of formula (1); 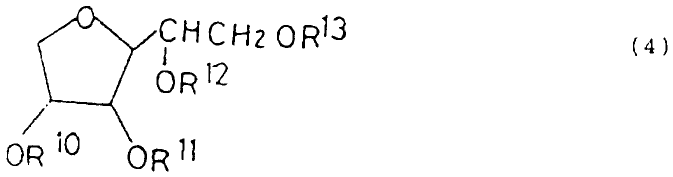 wherein R¹⁰, R¹¹, R¹² and R¹³ may be the same or different, and represent a hydrogen atom, a phenyl group, a methyl group or a group or formula (1) above, with the proviso that at least one of R¹⁰, R¹¹, R¹² and R¹³ represents a group of formula (1); 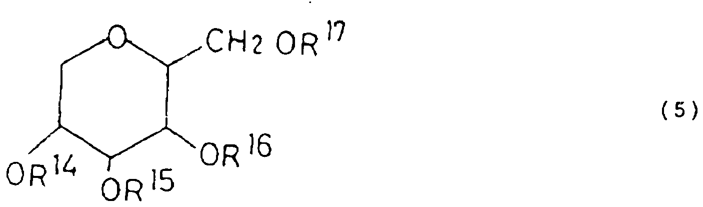 wherein R¹⁴, R¹⁵, R¹⁶ and R¹⁷ may be the same or different and represent a hydrogen atom, a phenyl group, a methyl group or a group of formula (1) above, with the proviso that at least one of R¹⁴, R¹⁵, R¹⁶ and R¹⁷ represents a group of formula (1); 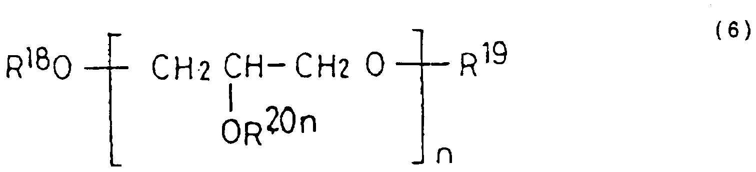 wherein n represents an integer of from 1 to 10; R¹⁸, R¹⁹ and R²⁰ⁿ may be the same or different, and represent a hydrogen atom, a phenyl group, a methyl group or a group of formula (1) above, with the proviso that at least one of R¹⁸, R¹⁹ and R²⁰ⁿ represents a group of formula (1).

7. A carrier for immobilizing microorganisms as claimed in any one of claims 2 to 6, wherein the epoxy compound is at least one of glycidol, glycerol diglycidyl ether, glycerol triglycidyl ether, polyglycerol polyglycidyl ether, polyethylene glycol diglycidyl ether, ethylene glycol diglycidyl ether, polypropylene glycol diglycidyl ether, sorbitol polyglycidyl ether, sorbitan diglycidyl ether, bis-(2,3-epoxycyclopentyl)-ether, vinylcyclohexanedioxide, butadienediepoxide, 1,2-bis-(2,3-epoxy-2-methylpropoxy)-ethane, and 1,1,3-tris-(2,3-epoxy-propoxy)-butane.

8. A carrier for immobilizing microorganisms as claimed in claim 2, wherein the N-methylol compound is at least one of dimethylolurea (DMU), methylated trimethylolmelamine (MTMM), dimethylolethylene urea (DMEU), dimethylolmethyl triazone (DMTr), dimethylolethyl triazone, dimethylolhydroxyethyl triazone, methylated dimethylol urone (DMUr), hexamethylol melamine (HMM), dimethylolpropylene urea (DMPU), dimethyloldihydroxyethylene urea (DMDHEU), tetramethylolacetylene diurea (TMADU), 4-methoxy-5-dimethylpropylene urea dimethylol compound (4MO, 5DM, PU), dimethylolmethyl carbamate (DMAC), dimethylolethyl carbamate, dimethytolhydroxyethyl carbamate, dimethylolhydroxyisopropyl carbamate, dimethyloldimethoxyethylene urea, dimethylolbutylene urea, dimethylol-5-hydroxypropylene urea, dimethylol urone, and tetramethylolethylene bistriazone.

9. A carrier for immobilizing microorganisms as claimed in claim 2, wherein the imidazolidinone compound is a compound represented by the following formula (7): 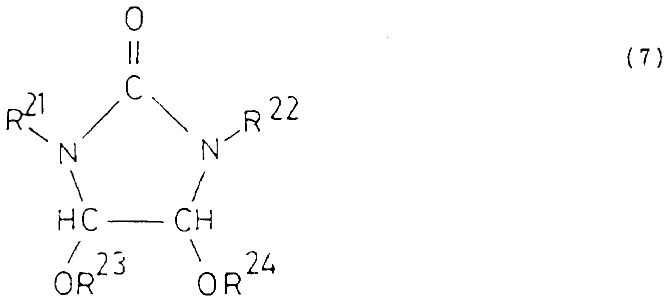 wherein R²¹ and R²² may be the same or different, and each represents a hydrogen atom, a lower alkyl group, an acyl group, or a group of -(CH₂-CH(CH₃)-O)ᵣ-H wherein r represents an integer of from 1 to 3, and R²³ and R²⁴ may be the same or different, and each represents a hydrogen atom, a lower alkyl group, or a lower acyl group.

10. A carrier for immobilizing microorganisms as claimed in claim 2 or 9, wherein the imidazolidinone compound is at least one of
4,5-dihydroxy-imidazolidinone,
1,3-dimethyl-4,5-dihydroxy-2-imidazolidinone,
1,3-diethyl-4,5-dihydroxy-2-imidazolidinone,
1,3-di(n-propyl)-4,5-dihydroxy-2-imidazolidinone,
1,3-di-isopropyl-4,5-dihydroxy-2-imidazolidinone,
1,3-di(α-dihydroxyethyl)-4,5-dihydroxy-2-imidazolidinone,
1,3-di(β-dihydroxyethyl)-4,5-dihydroxy-2-imidazolidinone,
1,3-dimethyl-4,5-dimethoxy-2-imidazolidinone,
1,3-dimethyl-4,5-diethoxy-2-imidazolidinone,
1,3-dimethyl-4,5-diisopropoxy-2-imidazolidinone,
1,3-dimethyl-4,5-diacetoxy-2-imidazolidinone,
1,3-di-(β-cyanoethyl)-4,5-dihydroxy-2-imidazolidinone,
1,3-di-(β-cyanoethyl)-4,5-dimethoxy-2-imidazolidinone,
1,3-di-(β-carbamoylethyl)-4,5-dihydroxy-2-imidazolidinone,
1,3-di-(β-carbamoylethyl)-4,5-dimethoxy-2-imidazolidinone,
1,3-di-(β-carboxyethyl)-4,5-dihydroxy-2-imidazolidinone,
1,3-di-(β-carboxyethyl)-4,5-dimethoxy-2-imidazolidinone,
1,3-di-(β-ethoxycarbonylethyl)-4,5-dihydroxy-2-imidazolidinone,
and
1,3-di-(β-ethoxycarbonylethyl)-4,5-dimethoxy-2-imidazolidinone.

11. A carrier for immobilizing microorganisms as claimed in claim 2, wherein the aldehyde compound is at least one of formaldehyde, glyoxal, acetaldehyde, a compound obtained by reacting cyclic urea with glyoxal, acrylic aldehyde, and a compound obtained by reacting acrylic amide with glyoxal.

12. A carrier for immobilizing microorganisms as claimed in claim 2, wherein the acetal compound is at least one of glycol acetal and pentaerythritol bisacetal.

13. A carrier for immobilizing microorganisms as claimed in claim 2, wherein the active vinyl compound is at least one of methacrylic acid hydroxy-propyltrimethyl-ammonium chloride, glycerol dimethacrylate, glycerol methacrylate acrylate, glycerol methacrylate alkenylate, diethylene glycol dimethacrylate, trimethylolpropane trimethacrylate, and zinc diacrylate.

14. A carrier for immobilizing microorganisms as claimed in claim 2, wherein the amidophosphazene is a compound represented by the following formula: $- {\left(N=P\right)}_{a} ⁢ {\left({NH}_{2}\right)}_{b} ⁢ {R}_{c}^{25}$
wherein R²⁵ represents a lower alkoxy group which may have a substituent, a phenoxy group which may have a substituent, a mono-lower alkylamino group, a di-lower alkylamino group, a represents an integer of 3 or more, and c represents an integer of 1 or more, with the proviso that b + c = 2a , and b/c≥ 1 .

15. A carrier for immobilizing microorganisms as claimed in claim 2 or 14, wherein the amidophosphazene is at least one of an amino-diethylamidophosphazene oligomer, a tetraamino-di-n-propoxycyclotriphosphazene, and pentaamino-monophenoxycyclotriphosphazene.

16. A carrier for immobilizing microorganisms as claimed in claim 2 or any of claims 6 to 15, wherein at least one of the epoxy compound having an epoxy group, the N-methylol compound, the imidazolidinone compound, the aldehyde compound having an aldehyde group, the acetal compound, the active vinyl compound having a vinyl group, the aziridinyl compound, the compound having a carboxyl group, the compound having an acyl group, the compound having an isocyanate group, the quaternary ammonium compound and the amidophosphazene compound is reacted with cellulose in an amount of from 3 to 60% by weight based on the weight of the cellulose.

17. A carrier for immobilizing microorganisms as claimed in any of claims 1 to 16,
wherein the cellulose is a porous cellulose.

18. A carrier for immobilizing microorganisms as claimed in any of claims 1 to 17,
wherein the cellulose has a pore diameter of from 30 to 2000 µm.

19. A method of converting nitrogen compounds in a liquid, which comprises introducing the carrier for immobilizing microorganisms as claimed in any of claims 1 to 18 into a liquid treatment tank under aerobic conditions or anaerobic conditions together with a liquid to be treated, and stirring the resulting mixture in the tank,there by converting the nitrogen compounds contained in the liquid to be treated.

20. A method according to claim 19, which comprises introducing the carrier for immobilizing microorganisms as claimed in any one of claims 1 to 18 into a nitrification treatment tank under aerobic conditions together with a liquid to be treated.

21. A method according to claim 19, which comprises introducing the carrier for immobilizing microorganisms as claimed in any one of claims 1 to 18 into a denitrification treatment tank under anaerobic conditions together with a liquid to be reacted.

22. A method according to claim 19, which comprises introducing the carrier for immobilizing microorganisms as claimed in any one of claims 1 to 18 into a nitrification treatment tank under aerobic conditions and a denitrification treatment tank under anaerobic conditions together with a liquid to be treated, stirring the resulting mixtures in the tanks, flowing the entire liquid to be treated from the nitrification treatment tank under aerobic conditions to the denitrification treatment tank under anaerobic conditions, thereby converting the nitrogen compounds contained in the liquid to be treated.

23. A method according to claim 19, which comprises introducing the carrier for immobilizing microorganisms as claimed in any one of claims 1 to 18 into a nitrification treatment tank under aerobic conditions and a denitrification treatment tank under anaerobic conditions together with a liquid to be treated, stirring the resulting mixture, flowing the entire amount of the liquid to be treated from the denitrification tank under anaerobic conditions to the nitrification treatment tank under aerobic conditions, and circulating part of the liquid to be treated between the denitrification treatment tank under anaerobic conditions and the nitrification treatment tank under aerobic conditions, thereby converting the nitrogen compounds contained in the liquid to be treated.

24. A method according to any one of claims 19 to 23, wherein the liquid to be treated is a potable water or a wastewater.

25. A method according to any one of claims 19 to 23, wherein the cellulose derivative is introduced into the treatment tank in an amount of from 3 to 100% by volume based on the volume of the treatment tank.
